(19) **Europäisches Patentamt**
**European Patent Office**
**Office européen des brevets**

(11) **EP 2 721 178 B1**

(12) **EUROPEAN PATENT SPECIFICATION**

(45) Date of publication and mention
of the grant of the patent:
**20.07.2016 Bulletin 2016/29**

(51) Int Cl.:
***C12Q 1/68*** *(2006.01)*

(21) Application number: **12738204.2**

(22) Date of filing: **15.06.2012**

(86) International application number:
**PCT/IB2012/053021**

(87) International publication number:
**WO 2012/172511 (20.12.2012 Gazette 2012/51)**

(54) **METHOD FOR THE PROGNOSIS OF BREAST CANCER BASED ON EXPRESSION MARKERS**

VERFAHREN ZUR PROGNOSE VON BRUSTKREBS BASIEREND AUF EXPRESSIONSMARKERS

MÉTHODE POUR LE PRONOSTIC DU CANCER DU SEIN SUR LA BASE DE MARQUEURS D'EXPRESSION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.06.2011 IT PD20110201**

(43) Date of publication of application:
**23.04.2014 Bulletin 2014/17**

(73) Proprietors:
• **Universita' Degli Studi Di Trieste**
**34127 Trieste (IT)**
• **University Of Dundee**
**Dundee DD1 4HN (GB)**

(72) Inventors:
• **DEL SAL, Giannino**
**I-34123 Trieste (IT)**
• **NAPOLI, Marco**
**I-90124 Palermo (IT)**
• **GIRARDINI BROVELLI, Javier Enrique**
**Rosario 2000 (AR)**
• **PIAZZA, Silvano**
**I-33053 Latisana (IT)**
• **THOMPSON, Alastair**
**Auchterarder PH3 1DF (GB)**

(74) Representative: **Gervasi, Gemma et al**
**Notarbartolo & Gervasi S.p.A.**
**Corso di Porta Vittoria 9**
**20122 Milano (IT)**

(56) References cited:
**WO-A2-2005/083429     WO-A2-2008/094678**

• **FIAMMA MANTOVANI ET AL: "The prolyl isomerase Pin1 orchestrates p53 acetylation and dissociation from the apoptosis inhibitor iASPP", NATURE STRUCTURAL & MOLECULAR BIOLOGY, vol. 14, no. 10, 1 October 2007 (2007-10-01), pages 912-920, XP055006064, ISSN: 1545-9993, DOI: 10.1038/nsmb1306**
• **FIAMMA MANTOVANI ET AL: "KeePin' the p53 Family in Good Shape", CELL CYCLE, vol. 3, no. 7, 1 July 2004 (2004-07-01), pages 903-909, XP055006066, ISSN: 1538-4101, DOI: 10.4161/cc.3.7.999**
• **LIM: "Interpretation of Pin-1 and VEGF-C expression in breast infiltrating duct carcinoma", ONCOLOGY REPORTS, vol. 22, no. 06, 14 October 2009 (2009-10-14), XP055006068, ISSN: 1021-335X, DOI: 10.3892/or_00000578**
• **XU G G ET AL: "Pin1 as an anticancer drug target", Journals on the Web DRUG NEWS AND PERSPECTIVES, September 2009 (2009-09), XP002658099, PROUS SCIENCE ESP ISSN: 0214-0934, DOI: 10.1358/DNP.2009.22.7.1414594 Retrieved from the Internet: URL:http://journals.prous.com/journals/servlet/xmlxsl/pk_journals.xml_summary_pr?p_JournalId=3&p_RefId=1414594&p_IsPs=N [retrieved on 2011-09-08]**
• **ALASTAIR M THOMPSON ET AL: "p53 transcriptional pathways in breast cancer: the good, the bad and the complex", THE JOURNAL OF PATHOLOGY, vol. 220, no. 4, 1 January 2009 (2009-01-01), pages 401-403, XP055006070, ISSN: 0022-3417, DOI: 10.1002/path.2674**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 721 178 B1

• **TROESTER MELISSA A ET AL: "Gene expression patterns associated with p53 status in breast cancer", BMC CANCER, BIOMED CENTRAL, LONDON, GB, vol. 6, no. 1, 6 December 2006 (2006-12-06), page 276, XP021023062, ISSN: 1471-2407, DOI: 10.1186/1471-2407-6-276**
• **JAVIER E. GIRARDINI ET AL: "A Pin1/Mutant p53 Axis Promotes Aggressiveness in Breast Cancer", CANCER CELL, vol. 20, no. 1, 1 July 2011 (2011-07-01), pages 79-91, XP055006071, ISSN: 1535-6108, DOI: 10.1016/j.ccr.2011.06.004**

Remarks:

The file contains technical information submitted after the application was filed and not included in this specification

**Description**

**Field of the invention**

The invention relates to a prognostic method for breast tumours based on the expression of the prolyl isomerase enzyme Pin1 combined with detection of the presence of missense mutations in the *TP53* gene and in particular to a *molecular signature* produced by the characteristic 10-gene expression associated with the Pin1/mutant p53 axis.

**State of the art**

Several evidences have shed light on how point mutations within the *TP53* gene represent crucial preconditions for tumour onset and transformation, and the formation of metastases. Mutations of the tumour suppressor gene *TP53* are among the most frequently genetic changes of human tumours (Soussi T. and Wiman K., 2007). Besides being responsible for sporadic cancers, in humans hereditary mutations in *TP53* cause the Li-Fraumeni syndrome, a disorder characterised by the early-onset of a wide range of cancers (Malkin D. *et al.,* 1990). This type of mutation causes p53 to lose its ability to bind wild-type protein responsive elements present on DNA, and in this way mutant p53 loses the capacity to perform its tumour-suppressor functions and may exert a dominant-negative effect on the wild-type protein. Furthermore, many mutant p53 proteins acquire *de novo* a series of functional activities which significantly contribute to the manifestation of various aspects of cancer progression. In particular, strong evidence has shed light on the ability of the mutated protein to promote cell migration and metastasis formation (Adorno M. *et* al., 2009; Caulin C. *et al.,* 2007; Muller P. *et al.,* 2009; Terzian T. *et al.,* 2008).

For the gain of new functions, it is essential that mutant p53 forms aberrant protein complexes perturbing the activity of the interacting protein partners. One example of how mutant p53 contributes to the occurrence of the cancer phenotype by influencing characteristics such as cell migration and invasion, or genomic instability, is the interaction of mutant p53 with the anti-metastatic factor p63 or with Mre11, and the consequent inactivation thereof (Adorno *et al.,* 2009; Song H. *et al.,* 2007).

Many *in vivo* studies have demonstrated that, in cancer cells, the efficient gain of new functions by mutant p53 is associated with the presence of high levels of the same protein (Song H. *et al.,* 2007; Soussi T. and Beroud C., 2001; Terzian T. *et al.*, 2008). This evidence supports the hypothesis according to which stress signals may activate mutant p53 via mechanisms similar to those necessary for stimulating the action of wild-type p53 (Song H. *et al.,* 2007; Terzian T. *et al.,* 2008). The mechanisms by which oncogenic signals trigger the activity of mutant p53 are, however, still little understood.

Among the factors potentially capable of representing a link between specific cell signals of tumoral transformation and the functions performed by mutant p53, a candidate particularly interesting is the prolyl isomerase enzyme, Pin1. This is an enzyme which converts the phosphorylation signals present on its substrates in conformational changes involving modulation of their functions (Lu K.P. and Zhou X.Z., 2007; Yeh E. and Means A.R., 2007). This enzyme has been recognised as a critical regulator of the activities exerted by the wild-type protein p53 in cells exposed to genotoxic stress (Mantovani F. *et al.,* 2007; Zacchi P. *et al.,* 2002; Zheng H. *et al.,* 2002). Despite Pin1 is essential for the development of wild-type p53 functions, *Pin1$^{-/-}$* mice do not develop tumours (Atchinson F.W. *et al.,* 2003). In human tumours, on the other hand, Pin1 is frequently overexpressed (Bao L. *et al,* 2004), and in mammary cells it has been shown to be capable of promoting tumoral transformation events which depend on the proteins Her2/Neu/Ras or Notch1 (Rustighi A. *et al.,* 2009; Wulf G. *et al.,* 2004).

However, it is not known how, during tumoral transformation, the role played by Pin1 in the physiological surveillance systems of the cell is altered, rendering it a crucial amplifier of the oncogenic functions of mutant p53.

*TP53* mutations represent, in particular, one of changes, which frequently characterise breast cancers (Langerod A. *et al.,* 2007).

In general, breast carcinoma in women represents not only the most frequently diagnosed type of cancer, but also the principal cause of death (Jemal A *et al.,* 2011), and the *basal-like* sub-type is the type thereof with the highest risk of recurrence. It is estimated that, worldwide, the number of women to whom it is diagnosed exceeds one million (Coughlin S. *et al.,* 2009).

Indeed, breast cancer is a disease characterised by strong heterogeneity from various viewpoints, ranging from the histological to that of the response to the therapeutic treatments or the mode of metastatic spread into different regions of the body (Prat A. *et al.,* 2011).

Over the years, progress in the field of diagnosis and in prevention programmes and the development of new therapeutic strategies have led to a reduction in mortality due to this type of cancer (Jemal A. *et al.,* 2009). Nevertheless, it has been estimated that, in the United States and Europe, more than 120,000 women die of this disease each year (Jemal A. *et al.,* 2009; La Vecchia C. *et al.,* 2010). This is partly explained by the fact that the intrinsic complexity of this cancer is not associated to a complete knowledge of its biology, and even less to the availability of markers for use in clinical practice which fully reflect its heterogeneity, and which allow an accurate prognosis to be made in each case and enable the likelihood of success of the treatments to be determined. These factors are essential for guiding the choice of treatment, and, when necessary, the development of new intervention strategies.

In current clinical practice, the diagnosis of breast carcinoma is made by means of histopathological tissue investigation. The prognosis and the choice of treatment depend on this type of investigation, on evaluation of clinical parameters, such as the dimensions of the tumour, its stage, the age of the patient, and invasion of the lymph nodes by cancer cells, and on the molecular characterisation of the tumour based on expression of hormone receptors, HER2, and of a number of proliferation markers. Although these represent standard prognostic methods, they are not always effective in predicting the progression of the disease. Nevertheless, the possibility of more and more accurate evaluation of risk factors and formulating more precise predictions of the clinical course and of the outcome of the disease could have a considerable impact in respect of the treatment of breast tumours, because it would improve patient stratification. This need has given rise to the idea of linking the tests normally carried out with more in-depth molecular characterisation of the tumour. A prognostic test for breast tumours, the MammaPrint®, based on analysis of the expression of more than 70 genes using cDNA MicroArray technology, has therefore been developed (and approved by the FDA), the value of which for prognostic purposes has been demonstrated by means of studies conducted by van't Veer *et al.* (van't Veer L. *et al.,* 2002).

Methods for determining the prognosis of a breast cancer are known in the prior art, for example in WO 2008/094678 or WO 2005/083429.

This is the context into which the present invention fits, offering the advantage of an analysis of high predictive value, from the prognostic viewpoint, but based on determination of a reduced number of parameters and on the use of simpler technologies, which can therefore be used immediately and with greater ease.

Summary

The present invention is based on experimental evidence proving that Pin1 promotes tumoral transformation in a mouse model of Li-Fraumeni syndrome and amplifies the oncogenic functions of mutant p53, whilst in human breast cancer the overexpression of Pin1 combined with the presence of missense mutations in *TP53* correlates with an unfavourable outcome of the disease.

In human cell lines derived from breast carcinoma, the inventors have found that the two proteins together promote a transcriptional programme which favours tumour aggressiveness and that 10 genes (forming a *molecular signature* associated with the Pin1/mutant p53 axis), of which 7 proved to be adequate for the prognostic evaluation, are involved in this programme.

In one aspect, the invention relates to a prognostic method for breast cancer, which comprises the detection of expression levels of a *molecular signature* associated with Pin1/mutant p53 by determination of the expression of the genes *DEPDC1* (Gene ID No 55635, isoform 1: RefSeq NM_001114120.1, NP_001107592.1; isoform 2: RefSeq NM_017779.4, NP_060249.2), *CPSF6* (Gene ID No 11052, RefSeq NM_007007.2, NP_008938.2), *C21orf45* (Gene ID No 54069, RefSeq NM_018944.2, NP_061817.1), *CENPA* (Gene ID No 1058, isoform 1: RefSeq NM_001809.3, NP_001800.1; isoform 2: RefSeq NM_001042426.1, NP_001035891.1), *FAM64A* (Gene ID No 54478, RefSeq NM_019013.2, NP_061886.2), *CCNE2* (Gene ID No 9134, RefSeq NM_057749.2, NP_477097.1), *BUB1* (Gene ID No 699, RefSeq NM_004336.3, NP_004327.1), *EPB41L4B* (Gene ID No 54566, isoform 1: RefSeq NM_018424.2, NP_060894.2; isoform 2: RefSeq NM_019114.3, NP_061987.3), *NCAPH* (Gene ID No 23397, RefSeq NM_015341.3, NP_056156.2), *WDR67* (Gene ID No 93594, isoform 1: RefSeq NM_145647.3, NP_663622.2; isoform 2: RefSeq NM_001145088.1, NP_001138560.1). The use of this *molecular signature* in a computer implemented method in particular also offers the advantage of stratifying patients with ER+ (oestrogen-positive) tumours. Indeed, by means of the method proposed by the inventors, it is possible to identify within this group of cases - which is typically considered homogenous - two different subgroups, which show significant differences in respect of the prognosis. The stratification permitted by this method could lead to a choice of treatment different from that currently operated in these cases, enabling identification of those patients within the group of ER+ cases who could or may not benefit from the combination of hormone treatment and chemotherapy.

In a further aspect, the invention relates to kits for carrying out the prognostic method for tumours on the basis of determination of the expression levels of the genes of the *molecular signature* associated with the expression of Pin1/mutant p53.

**Brief description of the figures**

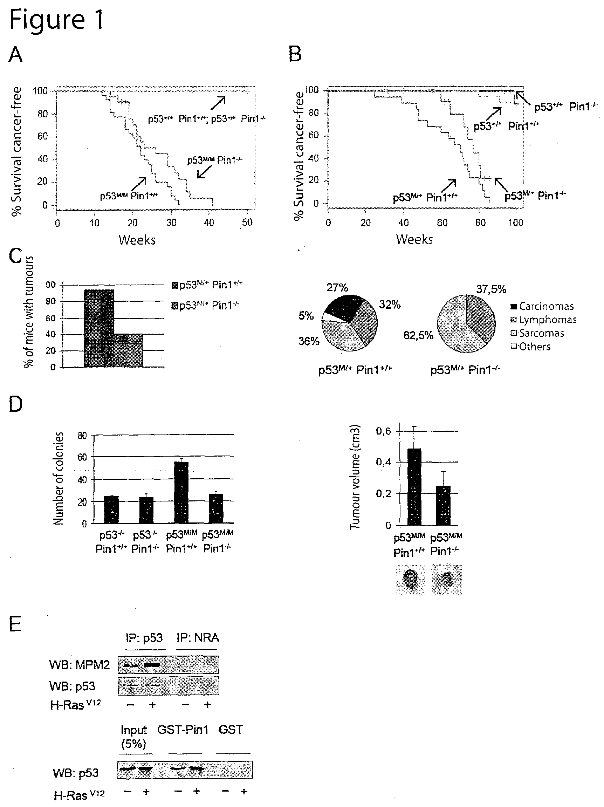

**Figure 1.** The figure shows how Pin1 increases tumorigenesis in mice "knockin" for mut. P53 **(A)** Kaplan-Meier curves of tumour-free survival in the mouse cohorts $p53^{M/M} Pin^{+/+}$ (n=27) and $p53^{M/M} Pin^{-/-}$ (n=22) ($X^2$=4, $P$=0.02, "long-rank" test of Mantel-Haenszel. **(B)** Kaplan-Meier curves of tumour-free survival in the mouse cohorts $p53^{M/+} Pin^{+/+}$ (n=19) and $p53^{M/+} Pin^{-/-}$ (n=17) ($X^2$=5.4, $P$=0.045, Mantel-Haenszel log-rank test). (A) and (B) $p53^{+/+} Pin^{+/+}$ (n=22) and $p53^{+/+} Pin^{-/-}$ (n=18). **(C)** Left: bar graph showing a percentage of animals developing tumours among mice presenting the indicated genotype. Right: pie graphs representing the percentage relative to the total various types of tumour present in $p53^{M/+} Pin^{+/+}$ and $p53^{M/+} Pin^{-/-}$ mice. **(D)** Left: bar graph representing growth, independently of anchorage to the substrate, of mouse embryonic fibroblasts (MEF) transduced with a construct for expression of H-Ras$^{V12}$. The error bars indicate the s.d. (n = 3), P relative to the MEFs $p53^{M/M} Pin^{+/+}$ < 0.01 in all cases (two-tailed $t$-test). Right: the bar graph represents the volume of the tumours originated by the indicated with H-Ras$^{V12}$-transduced MEFs, following the injection in NOD SCID mice. The volume was measured after animal sacrifice. The graph shows the mean volume and the s.d. of the tumours derived from groups of 5 mice for each cell type injected. P < 0.01 (two-tailed $t$-test). A few representative images of tumours are shown below. **(E)** Top: Western-blot analysis of the phosphorylated S-P or T-P sites (MPM-2 antibody) on normalised levels of mutant p53R172H immunoprecipitated from $p53^{M/M} Pin^{+/+}$ MEF cells infected with a construct for expression of H-Ras$^{V12}$ (+) or with the empty vector (-). NRA: non-related antibody. Bottom: Western-blot analysis of the pull-down assays conducted using the GST-Pin1 recombinant protein. The cell lysates used in the analysis were normalised as described above.

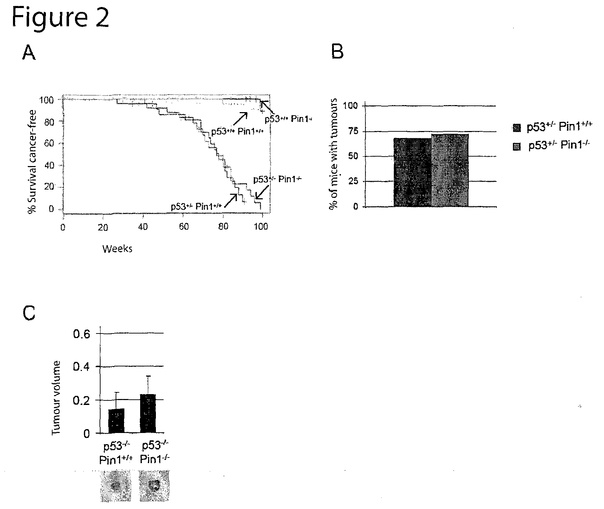

**Figure 2.** The figure depicts: **(A)** Tumor-free survival curves in the mouse cohorts $p53^{+/-} Pin^{+/+}$ (n=25) and $p53^{+/-} Pin^{-/-}$ (n=25). ($X^2$ = 0.2, $P$ = 0.68, Mantel-Haenszel log-rank test). $p53^{+/+} Pin^{+/+}$ (n=22) and $p53^{+/+} Pin^{-/-}$ (n=18). **(B)** Histogram graph showing, for each group, the percentage of mice which have developed tumours. (C) MEFs transduced with the vector for expression of H-Ras$^{V12}$ were injected subcutaneously into NOD SCID mice and, after sacrificing the animals, the volume of the generated tumours was measured. The graph shows the mean volume and the s.d. of tumours taken from 5 mice which had received injection of the cells of the type indicated. Two representative images of the tumours encountered are shown.

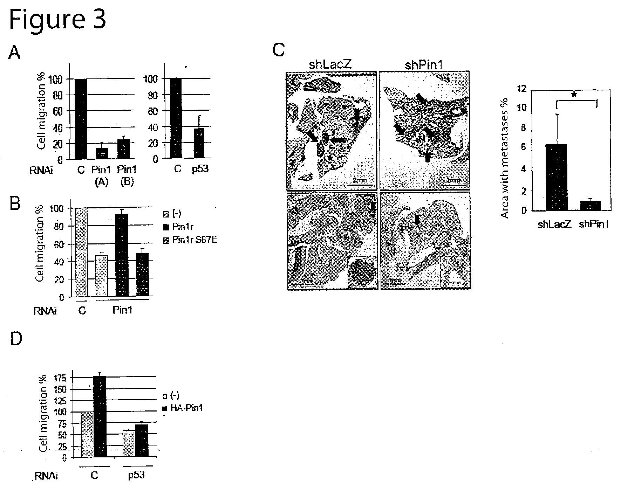

Figure 3. The figure shows the results which demonstrate that Pin1 amplifies the effects exerted by mutant p53 in favour of migration. (A) Transwell migration assays of MDA-MB-231 human cells lines, derived from breast tumours transfected with the indicated siRNAs (RNAi). The error bars indicate the s.d. (n = 3). The effects on migration of two different sequences of siRNA directed against Pin1 (Pin1 (A) and Pin1 (B)) were analysed. (B) Transwell migration assays of MDA-MB-231 cells transfected with a control siRNA (C) or with the siRNA Pin1 (A), following transduction of retroviral vectors for expression of the forms resistant to the siRNA Pin1 HA-Pin1r, HA-Pin1r S67E (catalytically inactive) or of the empty vector (-). The error bars indicate the s.d. (n = 3). (C) Lung colonization assays following injection into the caudal vein of Pin1-deplited MDA-MB-231 cells. Left: histological analysis of the pulmonary colonization. Haematoxylin and eosin staining of representative sections of the entire pulmonary lobes taken from mice inoculated with the shLacZ expression vector-infected (control) or with one for shPin1-infected cells (6 mice in each group), the arrows indicate a number of representative metastases (top image). Immunohistochemical detection of the expression of Pin1 (12.5x magnification) (images at the bottom). inserts show a detailed image of the area indicated by the arrows (200x magnification). Right: computer-aided assessment of percentage of lung tissue area occupied by metastases. The data are presented in histograms as average $\pm$ SEM (Mann-Whitney sum-rank test, $P$ = 0.009). **(D)** Transwell migration assays of MDA-MB-231 cells transfected with an siRNA specific for p53 or a control (C), following transduction of retroviral vectors pMSCV (-) or pMSCV HA-Pin1. The error bars indicate the s.d. (n = 3).

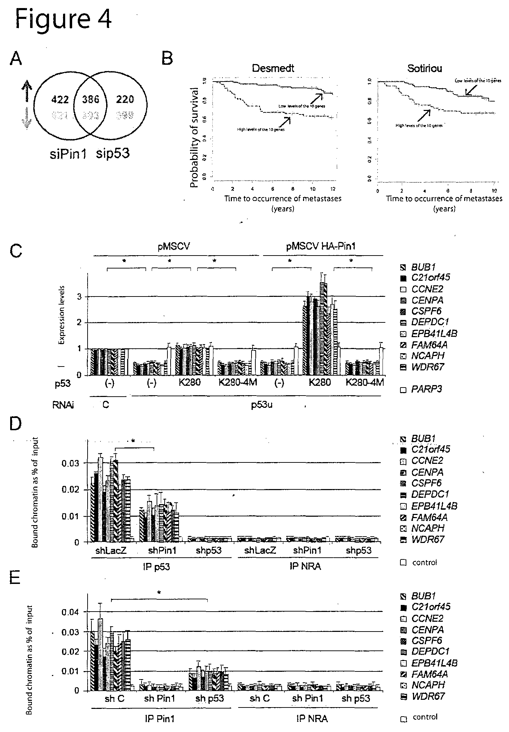

**Figure 4.** The figure shows that Pin1 and mutant p53 reprogram gene expression. **(A)** Venn diagram showing the genes simultaneously perturbed upon depletion of Pin1 or mutant p53 in MDA-MB-231 cells: up-regulated genes (upwardly-pointing arrow); down-regulated genes (downwardly-pointing arrow). **(B)** Kaplan-Meier survival curves based on the time range from diagnosis of the primary tumour to metastases in other regions of the body in patients with breast carcinoma classified on the basis of data for expression of genes belonging to the *molecular signature* associated with Pin1/mutant p53. Dotted line: the curve of cases in which the 10 genes are expressed at high levels; solid line: the curve of cases in which the 10 genes are expressed at low levels. Left: Desmedt data set (Desmedt C. et al., 2007) ($X^2$=25.6, $P$=4.1 x $10^{-7}$ , n=198, log-rank test). Right: Sotiriou data set (Sotiriou C. et al., 2006) ($X^2$=7.2, P=0.0074, n=179, log-rank test). **(C)** Histogram graph showing the variations in the levels of expression of genes belonging to the *molecular signature* associated with Pin1/mutant p53 and of an unaffected control gene (*PARP3*) (Gene ID 10039, isoform 1: RefSeq NM_001003931.2, NP_001003931.2; isoform 2: RefSeq NM_005485.4, NP_005476.3). The analyses were performed using qRT-PCR in MDA-MB-231 cells following co-transduction of the vectors pMSCV or pMSCV HA-Pin1 with the constructs coding for the p53 mutants indicated in the figure, or with the empty vector (-), used as the negative control (n=3, *: $P$ < 0.001, if the levels of expression in each gene of the *molecular signature* associated with Pin1/mutant p53 are compared under the various conditions indicated two-tailed $t$-test). (D) ChIP analysis of mutant p53-bound chromatin (IP p53) extracted from MDA-MB-231 cells following transduction with vectors for the expression of shRNA directed against endogenous Pin1 (shPin1), against mutant p53 (shp53) or against LacZ for negative control (shLacZ). The chromatin was immunoprecipitated with the DO1 antibody or with purified murine IgGs for negative control (NRA). The presence of the factor on the promoters was analysed by qRT-PCR and quantified as a percentage of the chromatin used in the input. (n=3, *: $P$ < 0.013 if the extent to which the factor is present on the promoter of each gene belonging to the *molecular signature* associated with Pin1/mutant p53 is compared under the conditions indicated, without considering the negative control, two-tailed *t*-test). (E) ChIP analysis of the Pin1-bound chromatin (IP Pin1) extracted from MDA-MB-231 cells following transduction with vectors for the expression of shRNA directed against endogenous Pin1 protein (shPin1), against mutant p53 (shp53) or against LacZ for negative control (shLacZ). The chromatin was immunoprecipitated with a polyclonal antibody directed against Pin1 or with purified rabbit IgGs for the negative control (NRA). The presence of the enzyme on the promoters was analysed as described in (D). The control in (D) and in (E) (the histograms in white) is represented by a region of the promoter of *DEPDC1* (from -2112 to -1909 respect to the sequence NM_032977.9), bound by neither mutant p53, nor by Pin1. From (C) to (E) the error bars indicate the s.d. (n = 3, two-tailed *t*-test).

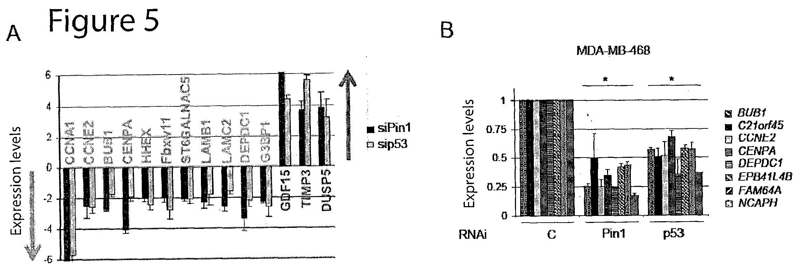

**Figure 5. (A)** Validation by qRT-PCR of the selected target genes of Pin1/mutant p53. The variations in the levels of gene expression are reported in terms of how many fold a gene is differentially expressed (*fold change*) in the MDA-MB-231 cells after the levels of Pin1 (siPin1) or of mutant p53 (sip53) have been depleted relative to what happens in the same type of cells transfected with the control siRNA. The error bars indicate the s.d. (n=3). **(B)** Effect produced by depletion of the levels of Pin1 or of mutant p53 in MDA-MB-468 cells on expression of the indicated target genes of Pin1/mutant p53. The mRNA levels were determined by qRT-PCR following transfection of the indicated siRNAs. The variations in the gene expression levels are reported in terms of how many folds a gene is differentially expressed following depletion of the levels of Pin1 (siPin1) or of mutant p53 (sip53) relative to the condition in which the cells were transfected with the control siRNA. The error bars indicate the s.d. (n=3. *: P value < 0.028 if the expression of each gene of the *molecular signature* associated with Pin1/mutant p53 transfected with the indicated siRNAs is compared with the control (two-tailed *t*-test)).

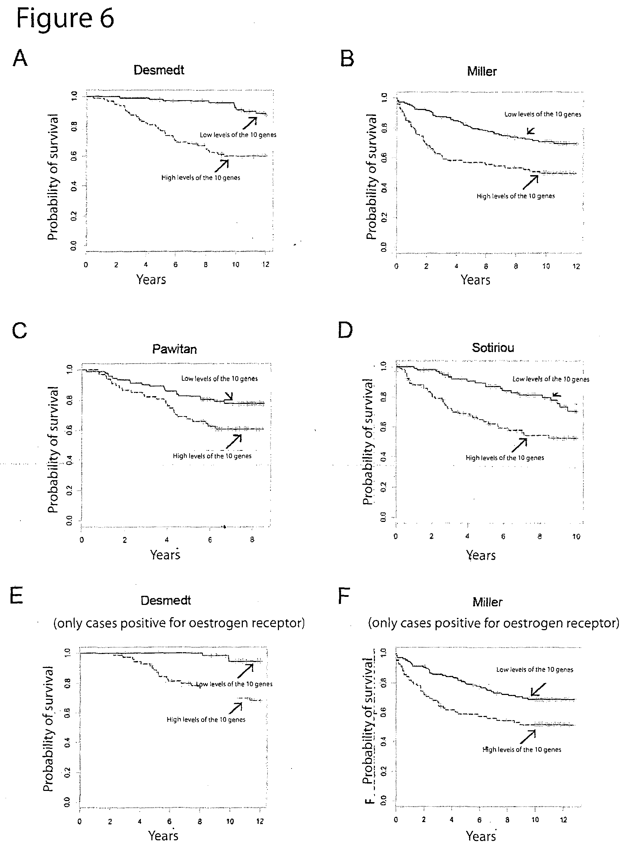

**Figure 6.** From **(A)** to **(D)** Kaplan-Meier analyses of the survival of breast-tumour patients classified on the basis of expression of the 10 genes of the *molecular signature* associated with Pin1/mutant p53. Dotted line: cases with elevated levels of expression of the 10 genes. Solid line: cases with low levels of expression of the 10 genes:

(A) Desmedt dataset ($X^2$=25.7, P=4.1 x$10^{-7}$, n=198, log-rank test);
(B) Miller dataset ($X^2$=9.7, P=0.002, n=251, log-rank test);
(C) Pawitan dataset ($X^2$=7.7, P=0.00557, n=187, log-rank test);
(D) Sotiriou dataset ($X^2$=11.6, P=0.00065, n=189, log-rank test).

**(E)** and **(F):** Kaplan-Meier analysis of the survival of patients with a breast tumour positive for the oestrogen receptor (ER+). Dotted line: cases with elevated levels of expression of the 10 genes. Solid line: cases with low levels of expression of the 10 genes:

(E) Desmedt dataset ($X^2$=14.3, *P*=0.00016, n=134, log-rank test);
(F) Miller dataset ($X^2$=8.6, *P*=0.00334, n=211, log-rank test).

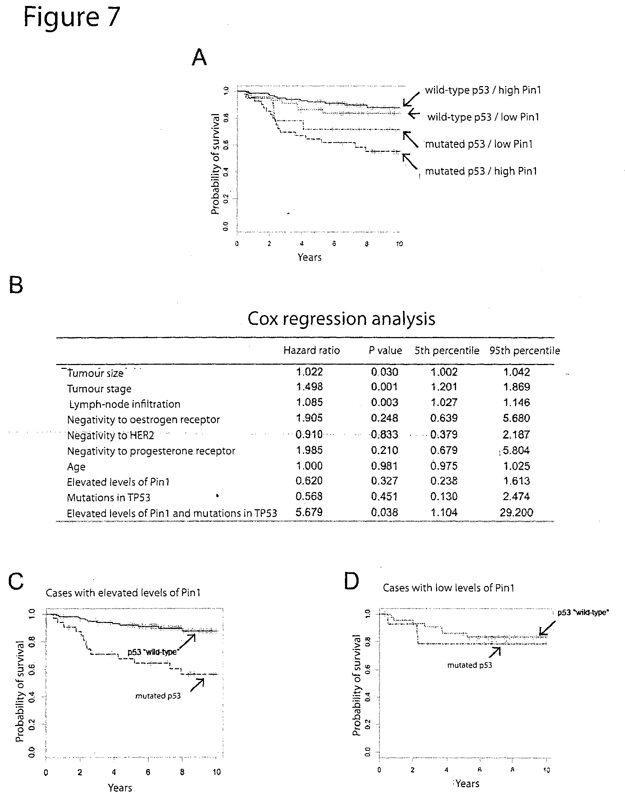

**Figure 7.** The figure shows that in breast carcinoma the presence of elevated levels of Pin1 influences the prognostic value of the presence of missense mutations in p53. **(A)** Kaplan-Meier curves of the survival of patients with a breast tumour (10-year limit of observation, clinical variable = OS) evaluated as a function of the combination of overexpression of Pin1 and of the presence of missense mutations in *TP53*. Low Pin1: tumours which do not overexpress Pin1, high Pin1: tumours which overexpress Pin1 ($X^2$=17.2, *P* < $10^{-4}$, n=212, log-rank test). **(B)** Multivariate analysis of the mortality risk. The multivariate analysis was performed on the same cohort of patients analysed in (A) using the Cox proportional hazards regression model. The relationship existing between survival and the presence of elevated levels of Pin1 and of missense mutations in *TP53* or other parameters used in clinical practice was shown. **(C)** Kaplan-Meier survival curves in cases of breast tumour with elevated expression levels of Pin1 (limit of observation 10 years, clinical variable = OS), as a function of the presence of p53, with missense mutations or wild-type ($X^2$=14.1, P < $10^{-4}$, n=143, log-rank test). **(D)** Kaplan-Meier survival curves in cases of breast tumour with low expression levels of Pin1 (limit of observation 10 years, clinical variable = OS), as a function of the presence of p53, with missense mutations or wild-type ($X^2$=0.4, *P*=0.548, n=69, log-rank test).

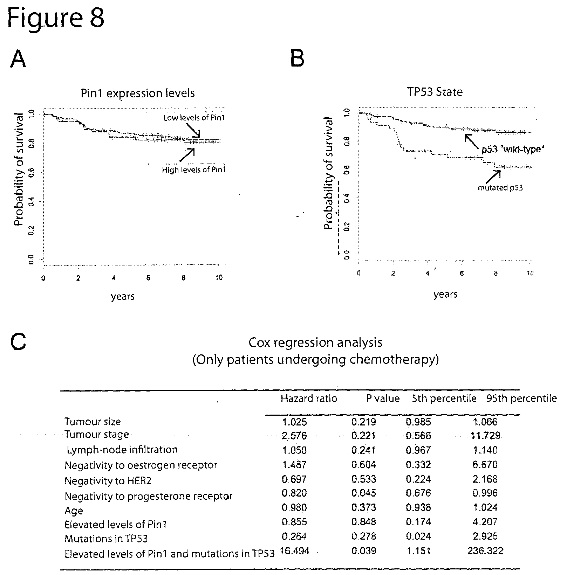

**Figure 8. (A)** Kaplan-Meier analysis of survival (OS) at 10 years in cases in which Pin1 was (solid line) or was not (dotted line) overexpressed. ($X^2$=0.0148506, *P*=0.903, n=212, log-rank test). **(B)** Kaplan-Meier analysis of survival (OS) at 10 years in cases in which *TP53* was mutated (solid line) or wild-type (dotted line). ($X^2$=21.43, P<0.001, n=212, log-rank test). **(C)** Multivariate analysis according to the Cox proportional hazards model carried out in patients undergoing anthracycline-based adjuvant chemotherapy, considered as a function of the overexpression of Pin1 and of the presence of missense mutations in *TP53* (n=66).

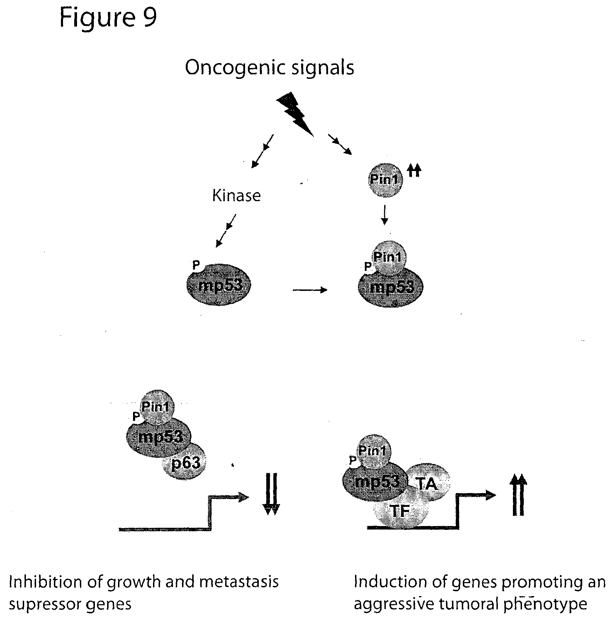

**Figure 9.** Diagram summarising the principal findings regarding the role of Pin1 and mutant p53 in favour of the aggressive tumour phenotype.

## Detailed description of the invention

*Definitions*

Gene *TP53* is used to mean the gene (Gene ID No 7157, RefSeq NM_000546.4, NP_000537.3) coding for the native (or *wild-type*) human protein p53 (GenBank accession No ABB80262).

The prolyl isomerase enzyme Pin1 is the protein (GenBank accession No AAC50492.1) coded by the gene *Pin1* (Gene **ID** No 5300, RefSeq NM_006221.2, NP_006212.1).

*Molecular signature* associated with the Pin1/mutant p53 axis is used to mean the group consisting of the following ten genes:

*DEPDC1* (DEP domain containing 1) identified by the Gene ID No 55635, *Homo sapiens* (isoform 1: RefSeq NM_001114120.1, NP_001107592.1; isoform 2: RefSeq NM_017779.4, NP_060249.2);

*CPSF6* (cleavage and polyadenylation specific factor 6, 68kDa) identified by the Gene ID No 11052, *Homo sapiens* RefSeq NM_007007.2, NP_008938.2; *C21orf45* (chromosome 21 open reading frame 45), identified by the Gene ID No 54069, *Homo sapiens* RefSeq NM_018944.2, NP_061817.1;

*FAM64A* (family with sequence similarity 64, member A) identified by the Gene ID No 54478, *Homo sapiens* RefSeq NM_019013.2, NP_061886.2;

*EPB41L4B* (erythrocyte membrane protein band 4.1 like 4B) identified by the Gene ID No 54566, *Homo sapiens* (isoform 1: RefSeq NM_018424.2, NP_060894.2; isoform 2: RefSeq NM_019114.3, NP_061987.3);

*NCAPH* (non-SMC condensin I complex, subunit H) identified by the Gene ID No 23397, *Homo sapiens* RefSeq NM_015341.3, NP_056156.2;

*WDR67* (WD repeat domain 67), identified by the Gene ID No 93594, *Homo sapiens* (isoform 1: RefSeq NM_145647.3, NP_663622.2; isoform 2: RefSeq NM_001145088.1, NP_001138560.1);

*CENPA* (centromere protein A) identified by the Gene ID No 1058, *Homo sapiens* (isoform 1: RefSeq NM_001809.3, NP_001800.1; isoform 2: RefSeq NM_001042426.1, NP_001035891.1);

*CCNE2* (cyclin E2) identified by the Gene ID No 9134, *Homo sapiens* RefSeq NM_057749.2, NP_477097.1;

*BUB1* (budding uninhibited by benzimidazoles 1 homologue (yeast)) identified by the Gene ID No 699, *Homo sapiens* RefSeq NM_004336.3, NP_004327.1.

"Score of the *molecular signature*" is used to mean the score associated with each sample, calculated using the value of expression of the genes of the *molecular signature* and the value of expression of three further genes used as a reference threshold and defined as "reference genes".

"Reference genes" is used to mean genes whose expression is comparable in intensity to the genes of the *molecular signature,* and the presence of which is constant in biopsy samples, such as for example:

*ERCC6* (excision repair cross-complementing rodent repair deficiency, complementation, group 6), identified by the Gene ID No 2074 *Homo sapiens* RefSeq. NM_000124.2, NP_000115.1;

*ADCY3* (adenylate cyclase 3) identified by the Gene ID No 109, *Homo sapiens* RefSeq. NM_004036.3, NP_004027.2;

*TUBGCP2* (tubulin, gamma complex associated protein 2) identified by the Gene ID No 10844, *Homo sapiens* RefSeq. NM_006659.2, NP_006650.1;

*SOX15* (sex determining region Y-box 15) identified by the Gene ID No 6665 *Homo sapiens* RefSeq. NM_006942.1, NP_008873.1;

*RPL10* (ribosomal protein L10) identified by the Gene ID No 6134 *Homo sapiens* RefSeq. NM_006013.3, NP_006004.2;

*DGCR14* (DiGeorge syndrome critical region gene 14) identified by the Gene ID No 8220 *Homo sapiens* RefSeq. NM_022719.2, NP_073210.1;

*KIAA0586* identified by the Gene ID No 9786 *Homo sapiens* RefSeq. NM_014749.3, NP_055564.3;

*ZBTB17* (zinc finger and BTB domain containing 17) identified by the Gene ID No 7709 *Homo sapiens* RefSeq. NM_003443.2, NP_003434.2;

*TFIP11* (tuftelin interacting protein 11) identified by the Gene ID 24144 *Homo sapiens* RefSeq. NM_001008697.1, NP_001008697.1;

*ZNF672* (zinc finger protein 672) identified by the Gene ID No 79894 *Homo sapiens* RefSeq. NM_024836.1, NP_079112.1;

*TXN2* (thioredoxin 2) identified by the Gene ID No 25828 *Homo sapiens* RefSeq. NM_012473.3, NP_036605.2.

The preferred reference genes are: *ERCC6, ADCY3, TUBGCP2.*

The *"molecular signature"* score is graded into three levels: low score, medium score and high score. The procedure and application of the *molecular signature* score are described in detail in what follows in the Description.

*Description*

Many studies performed using a mouse model knockin for mutant p53 have shown how, in order to be functionally active, mutant p53 requires the presence of an oncogenic context. With the scientific evidence underlying the present invention, one of the linking elements, which act as a bridge between the oncogenic signals and acquisition of the aggressive phenotype, is provided by demonstrating how, within the cancer cells, the enzyme Pin1 and mutant p53 are integrated into a molecular axis activated following phosphorylation of mutant p53 in dependence on specific phosphorylation sites.

This conclusion is confirmed by the results of research conducted and reported in detail below.

In particular, the following observations support the importance of this isomerase in the events leading to the transduction of oncogenic signals, and the onset of mutant p53 activity:

- in mice which express mutant p53, but not Pin1, tumoral transformation is significantly limited;
- the increase in tumorigenesis phenomena was proved to depend strictly on Pin1 in experiments conducted on MEF *p53*$^{M/M}$ cells transduced with H-Ras$^{V12}$;
- the presence of oncogenic signals in these cells has induced an increase in the expression of Pin1 and in parallel the phosphorylation of mutant p53 at S/T-P sites, thus leading the two proteins to interact.

On the basis of the results reported in detail below, it can be deduced that Pin1 is important in these model systems as a crucial linking element between the presence of oncogenic signals and the full activation of mutant p53, which translates as an increase in genomic instability and in the development of metastases.

It has also been observed that, in breast carcinoma cells, the presence of aberrant signals leads to the phosphorylation of the S/T-P sites of the mutant p53 protein, favouring recognition of the protein by Pin1. This event is necessary to trigger the effect of mutant p53 for promoting cell migration and invasion.

The effects of the Pin1/mutant p53 axis have significant clinical relevance, as demonstrated by series of studies conducted on a cohort of patients with breast carcinoma. From the analyses it is, in fact, emerged that the prognostic value of the presence of mutation in p53 is strengthened if combined with quantification of the levels of Pin1. The multivariate statistical analysis demonstrated that the simultaneous presence of elevated levels of Pin1 expression (quantification of the nuclear signal greater than 5, evaluated immunohistochemically using the "Quick score" method (Detre S. et al., 1995) and of missense mutations in *TP53* represents already by itself an independent and strong prognostic factor of the clinical outcome of the disease. The *TP53* status in tumours which overexpress Pin1 has in fact enabled better stratification of patients into groups characterised by a longer or shorter survival time. This effect has also been observed for a sample of patients receiving anthracycline-based adjuvant chemotherapy.

The data, therefore, indicate that the use of the prognostic method proposed by the inventors (but not part of the invention herein disclosed), based on quantification of the levels of Pin1 and on detection of the mutational status of *TP53,* could offer significant advantages in disease characterisation stages. Combined analysis of these two parameters, by contrast with what occurs by considering only the presence of mutations of the *TP53* gene, allows better patient stratification. Indeed, a critical aspect at the time of classification of the disease is typically the identification of the cases at elevated risk of recurrence and the ability to predict (by means of specific biomarkers) the response of patients to the treatments. This is an essential prerequisite for being able to implement the treatment strategies and guide future therapeutic choices. From this point of view, in particular, the method based on quantification of the levels of Pin1, and on the mutational status of *TP53,* allows differentiation among the various cases of breast carcinoma of the subgroup of patients who: i) have a lower likelihood of survival; ii) respond inadequately to the therapeutic interventions (and in particular to the anthracycline-based adjuvant chemotherapy).

As well as by quantification of the levels of the protein Pin1 and identification of the mutational status of *TP53,* the prognostic value of the Pin1/mutant p53 axis can also be evaluated by combining quantification of the levels of the proteins Pin1 and p53, elevated levels of which correlate with the presence of missense mutations in its gene (Goh A. M. et al., 2011), thus obtaining just as precise a prognostic method, but based on a unique investigative system, that is immunohistochemical analysis of both proteins.

Such investigations may be performed on fresh or frozen biopsy samples, or biopsy samples that have been fixed and embedded in paraffin, by identifying a signal using the known methods of immunochemistry and/or molecular biology, based on determination of nucleic acids, such as the Polymerase Chain Reaction (PCR) and analysis with the use of specific kits already in use for the protein p53 (for example the AmpliChip P53 test) and/or sequencing or other methods known to the person skilled in the art. The prognosis is therefore evaluated on the basis of a score derived by quantification of the signal identified for expression of the proteins Pin1 and p53.

Entering into the specific details of the assay, in the case wherein the parameters to be measured are the levels of expression of the Pin1 protein and of the mutational status of the *TP53* gene, the immunohistochemical analysis for Pin1 on biopsy samples in paraffin and sequencing of the *TP53* gene can preferably be performed on RNA extracted from the fresh or frozen biopsy sample.

The detection of the immunohistochemical labelling and the detection of a gene mutation constitute the signals to be acquired and measured in order to determine the expression levels of Pin1 and of the *TP53* gene mutational status.

For the immunohistochemical-type evaluation, the reagents for determination of the expression of Pin1 are specifically polyclonal and monoclonal anti-Pin1 antibodies advantageously labelled for identification purposes.

To identify the mutational status of the *TP53* gene specific oligonucleotides are necessary, consisting of DNA sequences (5'-3' forward and reverse primer) for the amplification reaction, such as the for example, the known oligonucleotides indicated below in Table 1:

Table 1: 5'-3' Forward and reverse primer for identification of the mutational status of the *TP53* gene

| Primer symbol | Sequence (sense 5'-3') | Region of pairing | Reference genome sequence RefSeq |
|---|---|---|---|
| RT2 | aatgtcagtctgagtcaggcccttctg SEQ ID NO:1 | 1383-1357 | NM_000546.4 |
| GIL | tgatgctgtccccggacgatattgaa SEQ ID NO:2 | 325-350 | NM_000546.4 |
| MP9ER | tctcccaggacaggcacaaacacg SEQ ID NO:3 | 1037-1014 | NM_000546.4 |
| E6E7F | tttgcgtgtggagtatttggat SEQ ID NO:4 | 797-818 | NM_000546.4 |
| MP6R | ttggcaaaacatcttgttgagggc SEQ ID NO:5 | 605-582 | NM_000546.4 |

Rather than by sequencing, the mutational status of *TP53* could alternatively and preferably be inferred by evaluating the protein levels of p53 (quantification of the nuclear signal greater than 5, using the "Quick score" method (Detre S. et al., 1995) by immunohistochemical means with polyclonal and monoclonal anti-p53 antibodies on the same biopsy samples of breast cancer on which the Pin1 expression evaluation is performed. Such assessment of the p53 protein is, then, to be preferred as can be carried out in combination with the determination of the expression of Pin1 protein.

The method for breast cancer prognosis based on the Pin1 expression in combination with the detection of the *TP53* gene mutational status or p53 protein expression comprises essentially the following steps of:

- acquiring an immunohistochemical signal of the Pin1 protein expression;
- measuring thereof and assigning to the sample a score by means of the Quick score method;
- acquiring the signal of the *TP53* gene mutational status by gene sequencing; or
- acquiring an immunohistochemical signal of the p53 protein expression;
- classifying the sample in the group to good (low score for protein(s) and no *TP53* gene mutations) or poor prognosis (high score for protein(s) and *TP53* gene mutation) when the score for Pin1 and p53 expression is equal or lower to 5 or higher than 5 or a mutation of the *TP53* gene is detected with a score for Pin1 expression equal or lower to 5 or higher than 5.

The immunohistochemical labelling is carried out as known to an expert in the field and comprises at least the following steps of:

- preparing sections of tissue sample to be analysed;
- treating thereof with a solution comprising a primary antibody consisting of a polyclonal or monoclonal anti-Pin1 antibody added with a biotin solution for reduction of non-specific labelling;
- incubating the sections with a secondary biotinylated antibody and afterwards with a labelling agent, for example a chromogenic agent such as diaminobenzidine.

In case the option to measure the expression levels of p53 is chosen, the sections are subjected to double labelling by treating the samples with a primary antibody consisting in a poly- or monoclonal anti-p53 antibody.

The detection of *TP53* gene mutational status can be performed by PCR.

For signal measurement and assignment of a score according to the Quick score method; are essential the following steps of:

- evaluation of the positivity to labelling with antibodies calculating the percentage of the positive cells and assignment of a score from 1 to 6: 0-4% =1; 5-19%=2; 20-29%=3; 40-59%=4; 60-79%=5 and 80-100%= 6;
- detecting the signal deriving from labelling and evaluating the mean intensity with assignment of a score from 0 to 3: no signal=0; weak labelling signal = 1; medium labelling signal = 2; strong labelling signal = 3; and
- calculation of the Quick score by multiplying the score on the positive cell percentage and score on the signal intensity.

On the basis of the proposed method, it is possible to subdivide patients with breast cancers into two categories having a better (80% without metastases at 10 years), or worse prognosis (40% without metastases at 10 years). The cases in the latter group are those in which the tumour presents overexpressed Pin1 and p53 (for both, the immunohistochemical score must be greater than 5) or those in which the tumour overexpresses Pin1 (immunohistochemical score greater than 5) and presents mutations in *TP53* (identified by sequencing).

This method of prognosis can be perfected by integrating the detection of the levels of Pin1 and of the mutational status of *TP53* and/or of the levels of the p53 protein with the analysis of the *molecular signature* associated with the Pin1/mutant p53 axis. This evaluation, which is based on detection of the expression levels of the genes of the *molecular signature,* not only enables the prognosis of the disease to be obtained, even without knowing the status of Pin1 and p53, but also allows stratification of patients with breast tumours positive for oestrogen receptors.

The levels of expression of the genes of this *molecular signature* can be monitored on fresh or biopsy samples or on nucleic acids extracted therefrom, by determination of their levels of mRNA by PCR. Consideration should also be given to the convenience of more rapid analysis by detection of the levels of the proteins expressed by these genes, to be performed by immunohistochemistry on biological samples fixed and embedded in paraffin. In this case, also, the prognostic evaluation can be performed on the basis of the score derived by quantification of the signal ascertained for expression of the genes and/or of the proteins expressed by these genes.

The data obtained by the inventors, in fact, also show that the combined action of Pin1 and mutant p53 favours an aggressive tumoral phenotype via introduction of a specific transcriptional programme. Among the targets of the overexpressed Pin1 and mutant p53 axis, 10 genes, which constitute a *molecular signature* associated therewith: *DEPDC1* (Gene ID No 55635), *CPSF6* (Gene ID No 11052), *C21orf45* (Gene ID No 54069), *CENPA* (Gene ID No 1058), *FAM64A* (Gene ID No 54478), *CCNE2* (Gene ID No 9134), *BULB1* (Gene ID No 699), *EPB41L4B* (Gene ID No 54566), *NCAPH* (Gene ID No 23397), *WDR67* (Gene ID No 93594), have been identified.

This *molecular signature* has been found to significantly correlate with the clinical outcome of the disease, as shown by analysis of four independent datasets relating to different breast cancers cases on which a Kaplan-Meier analysis was performed:

- Desmet dataset (Desmet. C., et al., 2007);
- Miller dataset (Miller L.D. et al.. 2005);
- Sotiriou dataset (Sotiriou C. et al., 2006);
- Pawitan dataset (Pawitan Y. Et al., 2005).

To arrive at a definition of this *molecular signature,* therefore, the inventors moved from analysis of global gene expression under conditions of silencing both Pin1 and mutant p53, to a human cell model of triple-negative breast carcinoma. They then verified the importance of the transcriptional programme induced by Pin1 and by mutant p53 in primary tumours via investigation of four independent series of data relative to more than 800 cases of breast cancer. From this analysis, it has been found that overexpression of the genes most regulated under conditions of silencing both Pin1 and mutant p53 (31 genes) was encountered in tumours of patients having a less favourable prognosis. Starting from this group of 31 genes, a *molecular signature* was obtained on the basis of the score of correlation of the expression of each gene with the clinical data, using a Cox proportional hazards model. See Table 2 for the classification of the genes thus obtained.

Table 2. Gene classification on the basis of the correlation between expression data and clinical data.

| *Gene symbol* | Gene ID No RefSeq | Score |
|---|---|---|
| *CPSF6* | 11052 NM_007007.2, NP_008938.2 | 9.65 |

(continued)

| Gene symbol | Gene ID No<br>RefSeq | Score |
|---|---|---|
| C21 orf45 | 54069<br>NM_018944.2, NP_061817.1 | 9.35 |
| CENPA | 1058<br>NM_001809.3, NP_001800.1 | 8.55 |
| FAM64A | 54478<br>NM_019013.2, NP_061886.2 | 8.06 |
| CCNE2 | 9134<br>NM_057749.2, NP_477097.1 | 7.44 |
| BUB1 | 699<br>NM_004336.3, NP_004327.1 | 5.56 |
| EPB41L4B | 54566<br>isoform 1: NM_018424.2, NP_060894.2; isoform 2: NM_019114.3, NP_061987.3 | 5.23 |
| NCAPH | 23397<br>NM_015341.3, NP_056156.2 | 5.18 |
| DEPDC1 | 55635<br>isoform 1: NM_001114120.1, NP_001107592.1; isoform 2: NM_017779.4, NP_060249.2 | 3.74 |
| WDR67 | 93594<br>NM_001145088.1, NP_01138560.1 | 3.61 |
| NMU | 10874<br>NM_006681.2, NP_006672.1 | 2.87 |
| FBP1 | 2203<br>NM_000507.3, NP_000498.2 | 2.47 |
| GFRA1 | 2674<br>isoform 1: NM_001145453.1, NP_001138925.1; isoform 2: NM_005264.4, NP_005255.1 | 2.16 |
| SRD5A1 | 6715<br>NM_001047.2, NP_001038.1 | 1.99 |
| FBXW11 | 23291<br>isoform 1: NM_033645.2, NP_387449.2; isoform 2: NM_033644.2, NP_387448.2; isoform 3: NM_012300.2, NP_036432.2 | 1.64 |
| IGFBP3 | 3486<br>isoform 1: NM_001013398.1, NP_001013416.1; isoform 2: NM_000598.4, NP_000589.2 | 1.64 |
| CHEK1 | 1111<br>NM_001274.4, NP_001265.2 | 1.53 |
| ST6GALNAC5 | 81849<br>NM_030965.1, NP_112227.1 | 1.38 |
| LAMC2 | 3918<br>NM_005562.2, NP_005553.2 | 1.19 |
| CCNA1 | 8900<br>isoform 1: NM_003914.3, NP_003905.1; isoform 2: NM_001111045.1, NP_001104515.1; isoform 3: NM_001111046.1, NP_001104516.1 | 1.08 |

(continued)

| Gene symbol | Gene ID No RefSeq | Score |
|---|---|---|
| TNIK | 23043<br>isoform 1: NM_015028.2, NP_055843.1; isoform 2: NM_001161560.1, NP_001155032.1; isoform 3: NM_001161561.1, NP_001155033.1; isoform 4: NM_001161562.1, NP_001155034.1; isoform 5: NM_001161563.1, NP_001155035.1 ; isoform 6: NM_001161564.1, NP_001155036.1; isoform 7: NM_001161565.1, NP_001155037.1; isoform 8: NM_001161566.1, NP_001155038.1 | 0.98 |
| PRSS12 | 8492<br>NM_003619.3, NP_003610.2 | 0.77 |
| HHEX | 3087<br>NM_002729.4, NP_002720.1 | 0.54 |
| CRISPLD2 | 83716<br>NM_031476.3, NP_113664.1 | 0.52 |
| G3BP1 | 10146<br>NM_005754.2, NP_005745.1 | 0.39 |
| SAMHD 1 | 25939<br>NM_015474.3, NP_056289.2 | 0.27 |
| PDLIM4 | 8572<br>isoform 1: NM_003687.3, NP_003678.2; isoform 2: NM_001131027.1, NP_001124499.1 | 0.18 |
| LAMB1 | 3912<br>NM_002291.2, NP_002282.2 | 0.17 |
| DKK3 | 27122<br>NM_015881.5, NP_056965.3 | 0.1 |
| LOC151162 | 151162<br>NR_024275.1 | 0 |
| PNMAL1 | 55228<br>isoform 1: NM_018215.3, NP_060685.2; isoform 2: NM_001103149.1, NP_001096619.1 | 0 |

From the gene classification based on association with the clinical data, genes having a score greater than 3 were selected, thus obtaining a *molecular signature* associated with the Pin1/mutant p53 axis, consisting of 10 genes *DEPDC1, CPSF6, C21 orf45, CENPA, FAM64A, CCNE2, BUB1, EPB41L4B, NCAPH, WDR67.*

The expression of these genes correlates significantly with the clinical outcome of the disease: in patients who expressed these genes at higher levels relative to the mean value for all patients, the time interval between diagnosis of the primary tumour and that of metastases in other body regions (*Time to Distant Metastasis,* TDM) was shorter (2-3 years depending on the dataset) and survival was reduced (*Overall Survival,* OS, 2-3 years depending on the dataset). Expression of these 10 genes also has prognostic value when considering exclusively the patients with breast tumours positive for the oestrogen receptor. Similar results were obtained by analysing the expression of a group of seven genes consisting of: *C21orf45, CPSF6, DEPDC1, EP841L48, FAM64A, NCAPH e WDR67* (not part of the invention).

The expression of the *molecular signature* associated with Pin1/mutant p53 can be determined by measuring, using PCR, the expression of the mRNA of the 7 or 10 genes of the *molecular signature* associated with Pin1/mutant p53 on fresh or frozen biopsy samples and of reference genes whose expression is of comparable intensity to genes of the *molecular signature,* and the presence of which is constant in biopsy samples. These genes may be selected from *ERCC6, ADCY3, TUBGCP22, SOX 15, RPL10, DGCR14, KIAA0586, Z8T817, TFIP*11*, ZNF672, TXN2.* The reference genes are preferably the three genes *ERCC6, ACCY3, TUBGCP2.* The reagents may be the primers necessary for amplification of the mRNA extracted from the test sample, which primers are presented below in Table 3:

Table 3: 5'-3' Forward and reverse primers for identification of the 10 genes of the *molecular signature* and of reference genes

| Gene symbol | Sequence (sense 5'-3') | Region of pairing * | RefSeq mRNA di (mRNA ID) |
|---|---|---|---|
| BUB1 forward | ATTCAAGCCACAGAGTGGAGCAG SEQ ID NO:6 | 1354-1376 | NM_004336.3 |
| BUB1 reverse | AGAACTTGTGTTGGCAACCTTATGTG SEQ ID NO:7 | 1398-1423 | NM_004336.3 |
| C21ORF45 forward | GCGACTCGCTGAGCTGGGTG SEQ ID NO:8 | 329-348 | NM_018944.2 |
| C21ORF45 reverse | CCCCGCGCAGCACAAAGTCT SEQ ID NO:9 | 461-480 | NM the_018944.2 |
| CCNE2 forward | TGAGCCGAGCGGTAGCTGGT SEQ ID NO:10 | 53-74 | NM_057749.2 |
| CCNE2 reverse | GGGCTGGGGCTGCTGCTTAG SEQ ID NO:11 | 133-152 | NM_057749.2 |
| CENPA forward | CTTCCTCCCATCAACACAGTCG SEQ ID NO:12 | 287-308 | NM_001809.3 |
| CENPA reverse | TGCTTCTGCTGCCTCTTGTAGG SEQ ID NO:13 | 462-483 | NM_001809.3 |
| CPSF6 forward | AGGGGCTGTTCCTGGTGGGG SEQ ID NO:14 | 734-753 | NM_007007.2 |
| CPSF6 reverse | GGCCCAGCTAGAGGAGGAGGC SEQ ID NO:15 | 887-907 | NM_007007.2 |
| DEPDC1 forward | TGGGTATTATCTGCCATGAAGTGCCT SEQ ID NO:16 | 853-878 | NM_017779.4 |
| DEPDC1 reverse | AGGTTGCAGCAAGCCCAAAATGT SEQ ID NO:17 | 1019-1041 | NM_017779.4 |
| EPB41L4B forward | CGACGGGACCGAAGTGAGCG SEQ ID NO:18 | 571-590 | NM_018424.2 |
| EPB41L4B reverse | CAGTGCGCAACCTGGGCAGA SEQ ID NO:19 | 692-711 | NM_018424.2 |
| FAM64A forward | CTCCAGGCTGCAGCTCGCTC SEQ ID NO:20 | 353-372 | NM_00119522 8.1 |
| FAM64A reverse | CAGCCGGGTGCTCTTCTGGC SEQ ID NO:21 | 519-538 | NM_00119522 8.1 |
| NCAPH forward | GAGGAGCCTGCCCCCTGTCA SEQ ID NO:22 | 2169-2188 | NM_015341.3 |
| NCAPH reverse | TGGGCCTCCTGCTGCTGACT SEQ ID NO:23 | 2325-2344 | NM_015341.3 |
| WDR67 forward | AGGCAACAAGGAGAGCGGCA SEQ ID NO:24 | 108-127 | NM_145647.3 |
| WDR67 reverse | AGCAGTCGCCTGTGCCATCA SEQ ID NO:25 | 240-259 | NM_145647.3 |
| ERCC6 for | TCTCTGTTTCCTTGTGGGCGCTC SEQ ID NO:26 | 106-128 | NM_000124.2 |
| ERCC6 rev | CACCCCACAGCAGAGGTGGACA SEQ ID NO:27 | 315-336 | NM_000124.2 |
| ADCY3 for | GTGGGCTGGCAGGTCTTCTTTG SEQ ID NO:28 | 704-725 | NM_004036.3 |
| ADCY3 rev | GGTGCTTGCGGTCAGCCATGTA SEQ ID NO:29 | 932-953 | NM_004036.3 |
| TUBGCP2 for | GACGTCACGCTCACTTCCGCCG SEQ ID NO:30 | 257-278 | NM_006659.2 |
| TUBGCP2 rev | TCAGCCCCATCTCCTCCGTGGA SEQ ID NO:31 | 413-434 | NM_006659.2 |
| *corresponding to the sequence of the mature mRNA (from 5' to 3' on the sense strand) | | | |

Therefore, the signal to be measured and acquired for the detection of the gene set forming the *molecular signature* is the expression of the gene sequence detected by amplification with the PCR on samples consisting of fresh biopsies of breast cancer or a nucleic acid extracted from the same according to methods known in the state of the art.
By quantification of the expression of these genes, it is possible to subdivide the patients with breast tumours into three categories, having a good, moderately good or poor prognosis.
Such a stratification is possible on the basis of a *molecular signature* score obtained by calculating the relative genes scores by evaluating, for each of the genes, the relative expression with respect to one of the reference genes, as shown in Table 4 below:

Table 4

| Molecular signature | Reference genes |
|---|---|
| FAM64A | ADCY3 |
| CENPA | ADCY3 |
| C21orf45 | ADCY3 |
| NCAPH | ADYC3 |
| BUB1 | ERCC6 |
| CCN2 | ERCC6 |
| WDR67 | ERCC6 |
| DEPDC1 | ERCC6 |
| CPSF6 | TUBGCP2 |
| EPB41L4B | TUBGCP2 |

In particular, the gene score of the *molecular signature* is calculated on the basis of the following formula:

$$Sg=\begin{cases} \log(Exp_i,base=2)-\log(Exp_r,base=2)< -0.5 \implies Sg=0 \\ \begin{matrix}\log(Exp_i,base=2)-\log(Exp_r,base=2) \geq -0.5 \\ \log(Exp_i,base=2)-\log(Exp_r,base=2) \leq 0.5\end{matrix} \implies Sg=1 \\ \log(Exp_i,base=2)-\log(Exp_r,base=2)> 0.5 \implies Sg=2 \end{cases}$$

where:

a) if a gene *i* of the *molecular signature* has an expression value $Exp_i$ of less than-0.5 on the base 2 logarithmic scale by comparison with the corresponding expression value $Exp_r$ of its reference gene r (equivalent to saying that there exists a difference of 0.5 RT-PCR cycles), this gene is attributed a gene score of zero.
b) if a gene i of the *molecular signature* has an expression value $Exp_i$ of higher than 0.5 on the base 2 logarithmic scale by comparison with the corresponding expression value $Exp_r$ of its reference gene r (equivalent to saying that there exists a difference of 0.5 RT-PCR cycles), this gene is attributed a gene score of two.
c) if a gene *i* of the *molecular signature* has an expression value $Exp_i$ intermediate to the two values of cases a) and b) by comparison with the corresponding expression value $Exp_r$ of its reference gene r, this gene is attributed a gene score of one.

The sum of the gene scores (Sg) thus obtained determines the value of the score for the *molecular signature* of the 10 genes (signature score $S_f$):

$$S_f = \sum S_g$$

and

- a score value of the *molecular signature* of the ten genes ($S_f$) lower than or equal to 5 (low score) is associated with a favourable prognosis (80% without metastases at ten years);
- a score value of the *molecular signature* of the ten genes ($S_f$) lower than or equal to 9 and higher than 5 (medium score) is associated with a less favourable prognosis (60% without metastases at ten years);
- a value higher than 9 (high score) is associated with a poor prognosis (50% without metastases at ten years).

Alternatively, expression of the *molecular signature* associated with Pin1/mutant p53 could be determined in biopsy samples fixed and embedded in paraffin by measuring the levels of the protein products of the 7 genes of the *minimum molecular signature,* and preferably of all 10 genes of the *molecular signature* associated with Pin1/mutant p53 by means of immunohistochemical analysis. In this case, the reagents are suitably labelled polyclonal and monoclonal antibodies against the proteins produced by the gene. In this case, also, the nuclear signal can be quantified using the "Quick score" method (Detre S. et al., 1995). The same procedures of evaluation previously described for determination of the levels of Pin1 and/or p53 are applied.

Therefore, the method for breast cancer prognosis based on the detection in a biopsy sample of a *molecular signature* consisting of the expression of a panel of genes comprises at least the steps of:

- acquiring a signal of expression of a panel of genes forming the *molecular signature DEPDC1* (Gene ID No 55635, isoform 1: RefSeq NM_001114120.1, NP_001107592.1; isoform 2: RefSeq NM_017779.4, NP_060249.2), CPSF6 (Gene ID No 11052, RefSeq NM_007007.2, NP_008938.2), *C21orf45* (Gene ID No 54069, RefSeq NM_018944.2, NP_061817.1), *FAM64A* (Gene ID No 54478, RefSeq NM_019013.2, NP_061886.2), *EPB41L4B* (Gene ID No 54566, isoform 1: RefSeq NM_018424.2, NP_060894.2; isoform 2: RefSeq NM_019114.3, NP_061987.3), *NCAPH* (Gene ID No 23397, RefSeq NM_015341.3, NP_056156.2), *WDR67* (Gene ID No 93594, isoform 1: RefSeq NM_145647.3, NP_663622.2; isoform 2: RefSeq NM_001145088.1, NP_001138560.1), *CENPA* (Gene ID No. 1058, isoform 1: RefSeq NM_001809.3, NP_001800.1; isoform 2: RefSeq NM_001042426.1, NP_001035891.1), *CCNE2* (Gene ID No 9134, RefSeq NM_057749.2, NP_477097.1), *BUB1* (Gene ID No 699, RefSeq NM_004336.3, NP_004327.1) or of the proteins expressed thereby;
- acquiring for comparison a signal of expression of a panel of reference genes consisting of at least *ERCC6* (Gene ID 2074, RefSeq. NM_000124.4, NP_000115.1), *ADCY3* (Gene ID 109, RefSeq NM_004036.3, NP_004027.2), *TUBGCP2* (Gene ID 10844, RefSeq. NM_006659.2, NP_006650.1) or of the proteins expressed thereby;
- classifying the sample in the group of good prognosis, medium or poor prognosis on the basis of a score obtained by the sum of the gene scores (Sg) of each gene calculated comparing the relative expression of each gene of the *molecular signature* with the expression of the reference genes

$$S_f = \sum S_g$$

where to a value of $S_f$ less than or equal to 5 (low score) is associated a better prognosis, to a value of $S_f$ less than or equal to 9 and higher than 5 (medium score) is associated an intermediate prognosis, and to a value of $S_f$ higher than 9 (high score) is associated a less favorable prognosis; or

- classifying the sample in the group of good or poor prognosis (score equal to or lower than 5 or higher than 5) on the base to the Quick score, in the case the expression of the proteins expressed by the genes is measured.

The kit for performing the prognostic method that is the object of the invention, based on determination of the expression levels of the genes of the *molecular signature,* and/or of the proteins expressed thereby comprises at least:

- a specific reagent for determination of the expression of the proteins expressed by 10 genes of the *molecular signature,* consisting of a polyclonal or monoclonal antibody; and/or
- oligonucleotides for the amplification of 10 genes of the *molecular signature,* and of one or more reference genes derived on the basis of the reference sequences thereof, or from their complementary sequences;
- a leaflet for the instructions.

Molecular characterisation of the tumour by detecting the *molecular signature* of 10 genes represents a reliable, sensitive and low-cost system, enabling prediction of the outcome of the disease, the risk of recurrence and the response of patients to the treatment (especially to the anthracycline-based adjuvant chemotherapy), especially in *basal-like* cases. To date, these represent a challenge in terms of treatment because they constitute a subgroup of breast tumours comprising predominantly triple-negative cases, defined as such because they do not express the hormonal receptors and do not overexpress HER2, have five-year survival rates very low, and are characterised by a high incidence of recurrences, which occur in spite of the adjuvant chemotherapy.

EXPERIMENTAL PART

**Materials and methods**

Cell cultures and constructs

MDA-MB-231 (p53R280K) and MDA-MB-468 (p53R273H) are human cells derived from breast carcinomas of the triple-negative type. SK-BR-3 (p53R175H) are human cells derived from breast tumours which overexpress HER2. All the cell lines were kept in culture in Dulbecco's modified Eagles Medium (DMEM) to which 10% FBS, penicillin (100 IU/ml) and streptomycin (100IU/ml) had been added. The MEF cells were generated by crossing mice of the appropriate genotype and recovering the cells from the embryos at 13.5 dpc, as described (Zacchi et al., 2002). pLPC and pLPC-H-Ras$^{V12}$ were kindly provided by M. Serrano (Efeyan et al. 2009); pGEX-Pin1 for the expression of GST-Pin1 and pGEX used as a control have already been described (Zacchi et al., 2002). pcDNA3-p53K280 and pcDNA3-p53K280-4M were created by site-specific mutagenesis, starting from the described constructs pcDNA3-p53 and pcDNA3-Mp53 (Zacchi et al., 2002). pMSCV-HAPin1 was generated by sub-cloning the specific coding sequence for Pin1 from the construct pcDNA3-HAPin1 (Mantovani et al., 2007) in the vector pMSCV. pMSCV-HAPin1r and pMSCV-HAPin1r S67E were generated by introducing, for site-specific mutagenesis of the constructs pcDNA3-HAPin1 and pcDNA3-HAPin1 S67E (Rustighi et al., 2009), silent mutations (G300A, G303A and T309C, relative to the sequence NM_006221.2) within the target region of the siRNA (A) specific to Pin1 and by then subcloning the mutagenically altered regions in the vector pMSCV. pLPC-p53K280 and pLPC-p53K280-4M have been created by subcloning the specific coding regions for p53K280 and p53K280-4M respectively, in the vector pLPC containing a HA tag.

The construct pSRshPin1 was created by cloning the double-stranded siRNA (A) specific to Pin1 (see below) in the vector pSuper Retro (Rustighi et al., 2009). The plasmid pSRshp53 was kindly provided by R. Agami (Drost J et al., 2010). The vector pSRshLAcZ has been described in Rustighi et al., 2009.

The lentiviral vector coding for the reporter gene of firefly luciferase used in assays to study the development of metastases in vivo, was described in Adorno et al., 2009.

Where not specified, the base vectors (commercially available) employed to create the various constructs were used as control vectors in the various experiments. The siRNA are given in Table 5.

Table 5: siRNA sequences used

| siRNA | Sequence (Sense, 5'-3') | Target gene | Species |
|---|---|---|---|
| Pin1 (A) | CGGGAGAGGAGGACUUUGA SEQIDNO:32 | Pin1 | *H. sapiens* |
| Pin1 (B) | GCCAUUUGAAGACGCCUCG SEQIDNO:33 | Pin1 | *H. sapiens* |
| p53 | GACUCCAGUGGUAAUCUAC SEQIDNO:34 | Tp53 | *H. sapiens* |
| p53u | GGUGAACCUUAGUACCUAA SEQIDNO:35 | Tp53 | *H. sapiens* |
| Controllo (LacZ) | GUGACCAGCGAAUACCUGU SEQIDNO:36 | Beta-D-galattosidasi | *E. coli* |

Mouse strains and analyses conducted in mice

The mouse cohorts used in this study originated from crossing *p53$^{M/+}$* or *p53$^{+/-}$* mice with *Pin1$^{+/-}$* mice, all having a genetic background of the type C57BL/6. The animals' genotype was characterised by analysing the polymerase chain reactions (PCR), as previously described (Atchinson F.W. et al., 2003; Lang G.A. et al., 2004). Animal showing signs of disease or the clear presence of tumours during the observations were sacrificed. The organs removed from them were kept for 24 hours in a solution of PBS, with 10% formalin for fixing. The tissues were embedded in paraffin and cut into slices 5μm thick. Before being subjected to pathological analysis, they were treated to eliminate the paraffin residues and stained with haematoxylin and eosin. The procedures involving animals were performed in accordance with the institutional guidelines, which had been drawn up with regard for the legislation and the relevant international policies (UKCCCR, 1989).

## Assays of transformation and of tumorigenesis *in vivo*

For the assays, murine embryo fibroblasts (MEF) from the mice of the strains *p53$^{M/M}$ Pin1$^{+/+}$, p53$^{M/M}$ Pin1$^{-/-}$, p53$^{-/-}$ Pin1$^{+/+}$* and *p53$^{-/-}$ Pin1$^{-/-}$*, which had undergone a low number of passages in culture, were used. The cells infected with the vector pLPC H-Ras$^{V12}$ or with its empty vector were resuspended in DMEM, to which had been added 10% foetal bovine serum, penicillin (100 IU/ml) and streptomycin (IU/ml), and containing 0.3% agarose. The cells were plated at a density of 1 x10$^5$ per plate 60 mm in diameter, on the surface of the layer of medium, presenting the same characteristics as the previous one, with the exception that it contains 1% agarose. After culturing for 14 days, the number of colonies reaching at least 100 $\mu$m in diameter were counted. All the experiments were conducted in triplicate using at least two different clones for genotype. To verify the tumorigenesis capacity of the MEFs, infected with the construct expressing H-Ras$^{V12}$, $1 \times \times 10^6$ cells were injected subcutaneously, and bilaterally in NOD SCID mice. After two weeks, the tumour volume was measured using a calliper, as described previously (Rustighi A. et al., 2009).

## Assays of migration and invasiveness

For the cicatrisation assays, the MDA-MB-231 transfected cells were seeded in 35 mm in diameter plates and cultured until 90% confluence was achieved. The cells were scraped from the surface using a 200-$\mu$l needle tip. Closure of the lesion was monitored for 36 hours. For the transwell migration assays, PET inserts of 24 wells (pore dimension 8.0 $\mu$m, Falcon) were used. The assays of invasiveness were conducted in PET inserts of 24 wells (pore dimension 8.0 $\mu$m, Falcon), using Matrigel®-coated filters.

## Assays to study the development of metastases *in vivo*

For the assays of pulmonary colonisation, cells of human MDA-MB-231 cell lines derived from breast tumours were co-transduced with a lentiviral vector coding for the reporter gene of firefly luciferase and with pSR shLacZ or pSR shPin1 (six animals in each group). 10$^6$ cells were resuspended in 100 $\mu$l of PBS and inoculated into the caudal vein of SCID mice. For *in vivo* visualisation, at different times from the cell inoculation bioluminescence images were acquired using a "cooled charge-coupled" acquisition device mounted above the camera obscura used for lodging the sample (IVIS Lumina II Imaging System; Caliper Life Sciences, Alameda, CA). 10 minutes prior to acquisition of the images, a solution containing 150 mg/kg of D-luciferin (Caliper Life Sciences) in PBS was administered i.p. to the anaesthetised animals. The histological analyses on samples of pulmonary tissue embedded in paraffin and stained with haematoxylin and eosin were conducted one month after injection of the MDA-MB-231. For the computerised analysis of the pulmonary tissue, the area covered by the metastatic foci, detectable in the pulmonary lobe sections, was measured. The parameter was calculated using a Leica DM200 microscope, fitted with a Leica DFC295 digital colour camera and Leica Application Suite (LAS) V3 software. The values for the total area and for the sum of the areas were, finally, compared with the value for the area of the whole pulmonary lobe. The expression of Pin1 was analysed by immunohistochemistry (IHC) using a monoclonal antibody (G-8, sc-46660, Santa Cruz) against this enzyme.

## Analysis of gene expression

The total RNA was extracted using Trizol (Invitrogen) and then subjected to treatment with DNasi-I (Ambion). For the microarray experiments, the mRNAs of three different biological samples for each group (siPin1 or sip53) were used for hybridisation of the Affymetrix GeneChip Human Genome U133A 2.0 array. In the experiments of quantitative RT-PCR, the mRNA was transcribed using the Superscript III (Invitrogen) system. The Real Time PCR was performed using a SYBR Green PCR master mix and an Applied Biosystems StepOne Plus thermal cycler. The sequences of the primers (sense 5'-3') for analysis of gene expression using qRT-PCR for the 10 genes of the *molecular signature,* and for the three control genes are presented in the previous Table 3, and correspond to the sequences: BUB 1 for SEQIDNO:6; BUB1 *rev* SEQIDNO:7; C21ORF45 *for* SEQIDNO:8; C21ORF45 *rev* SEQIDNO:9; CCNE2 *for* SEQIDNO:10; CCNE2 *rev* SEQIDNO:11; CENPA for SEQIDNO:12; CENPA *rev* SEQIDNO:13; CPSF6 *for* SEQIDNO:14; CPSF6 *rev* SEQIDNO:15; DEPDC1 *for* SEQIDNO:16; DEPDC1 *rev* SEQIDNO:17; EPB41L4B *for* SEQIDNO:18; EPB41L4B *rev* SEQIDNO:19; FAM64A *for* SEQIDNO:20; FAM64A *rev* SEQIDNO:21; NCAPH *for* SEQIDNO:22; NCAPH *rev* SEQIDNO:23; WDR67 *for* SEQIDNO:24; WDR67 *rev* SEQIDNO:25; ERCC6 *for* SEQIDNO:26; ERCC6 *rev* SEQIDNO:27; ADCY3 for SEQIDNO:28; ADCY3 *rev* SEQIDNO:29; TUBGCP2 *rev* SEQIDNO:30; TUBGCP2 for SEQIDNO:31.

Analysis of the microarray data

Annotations relating to gene expression and pre-processing: the intensity of the rough signals of gene expression was normalised at the level of the probe using the RMA algorithm, as previously described (Irizarry R.A. et al., 2003). The analysis at a low level of the microarray data was performed in an R/Bioconductor environment using, in particular, the limma and affy packages for microarray (http://www.R-project.org). The datasets publicly accessible used (GSE7390, GSE2990, GSE3494, GSE1456) were downloaded from the Gene Expression Omnibus web site (GSE7390, GSE2990, GSE3494) or from the web site of the authors of the study who produced them (GSE1456, http://www.mebki.se/-yudpaw/). The gene annotations relating to all the platforms considered were obtained from R-Bioconductor metadata packages (Gentleman R.C. et al., 2004). The analysis of differential expression of the genes of all the data sets considered in this study was conducted using functions and methods implemented in R/Bioconductor (Gentleman R.C. et al., 2004; Smyth G.K., 2004). In short, a fixed-effect linear model was individually applied to each gene to estimate the differences in expression between the groups of samples to be compared. To moderate the standard errors of the values of M, an empirical Bayes approach was used (Smyth, 2004). Finally, for each gene analysed, the moderated t-statistics, the log-odd ratio of differential expression (B-statistics), and the raw and adjusted P values were obtained (FDR control by means of the method of Benjamini and Hochberg).

Analysis of survival and microarray data for individuation of the *molecular siqnature.*

To verify the presence or otherwise of an association between expression of the 31 genes shown above in Table 2, concurrently more repressed under the two different experimental conditions analysed, and the survival time of patients with breast carcinoma (Desmedt et al., 2007), the Cox proportional hazards model was used. In particular, this model (Wald statistics) was applied to the expression of each gene to test its association with the survival data, so as to produce a coefficient or score of association with the clinical data. From classification of the genes based on association with the clinical data, those genes having a score of more than 3 were selected, thus obtaining a *molecular signature* consisting of 10 genes. To verify the correlation existing between the 10-gene *molecular signature* and the clinical data in cases of breast carcinoma, a Mantel Haenszel test was performed on the other normalised datasets used (GSE2990, GSE3494, GSE1456) (survival R package).

From the expression matrix for each dataset, the expression values for the genes forming the 10-gene signature were extracted by the use of the identifying univocal probe-set, thus obtaining a sub-matrix of expression. This matrix is composed of m rows/genes per n columns/samples, with m ranging from one to ten and n, ranging from 1 to the number of samples present in the study. To assign the same weight to each gene, the genes were normalised using the following procedure:

$$SX_{i,j} = \frac{X_{i,j} - \overline{V_j}}{mad(V_j)}$$

therefore for each row/gene *j*, yielding:

$$SV_j = \{SX_{j,1},\ SX_{j,2},\ \ldots,SX_{j,n}\}$$

$SV_j$ = standardised vector of gene expression, representing the values of gene expression standardised for a specific gene/row j for all the samples of the dataset (n);
$V_j$ = vector of gene expression, representing the values of gene expression for a specific gene/row *j* for all the samples of the data set (n). $V_j = \{X_{j,1},\ X_{j,2},...,X_{j,n}\}$;
$\overline{V_j}$ = estimated mean for a specific expression vector $V_j$ for a specific gene/row *j*. mad($V_j$)= median deviation for a specific expression vector $V_j$ for a specific gene/row *j*.

To measure the contribution of each gene to the 10-gene signature, a virtual gene (VG) vector is calculated by taking the average of each contribution of each gene starting from the standardised gene expression vector for all genes/rows m.

$$VG = \{VG_1, VG_2, \ldots, VG_n\} \text{ where } VG_k = \frac{1}{m}\sum_{j=1}^{m} SX_{j,k}$$

Finally, a sample k was defined as a "high-level 10-gene signature" if the value $VG_k$ was greater than the median value of the vector for the virtual gene VG, whereas a sample k was defined as a "low-level 10-gene signature" if the value $VG_k$ was less than the median value of the vector for the virtual gene VG:

High level: $VG_k$ > median VG,
Low level: $VG_k$ < median VG.

The survival curves were constructed using the Kaplan-Meier method (survival package). Thus with the log-rank test, the differences in expression of the 10 genes of the *molecular signature* were analysed (as described above) relative to the duration of the time interval between first appearance of the primary tumour, and that of metastases in other regions of the body (if the data was available), or relative to survival.

<u>Chromatin immunoprecipitation</u>

Chromatin was immunoprecipitated using the p53 DOI antibody (Santa Cruz) or a polyclonal antibody against Pin1 (Zacchi P. et al., 2002). The negative controls used were purified IgGs from rabbit or mouse serum. The co-immunoprecipitated DNA was analysed through Real Time PCR using a StepOne Plus thermal cycler (Applied Biosystems) and a SYBR Green Universal PCR master mix (Applied Biosystems). The presence of the factor on chromatin of the promoters analysed was calculated as a percentage of the immunoprecipitated quantity of chromatin used as the input, using the $2^{-\Delta Ct}$ method. The primer sequences are presented below in Table 6:

Table 6: primer 5'-3' for the 10 genes of the *molecular signature*

| Gene symbol | Sequence | Region of pairing | Reference genome sequence |
|---|---|---|---|
| *BUB1* (+) for | GGGCTGCCTCACACCCGTTT SEQIDNO:37 | 8105-8124 | NG_012048.1 |
| *BUB1* (+) rev | ACACCTGCCCTTCCAGTGGG SEQIDNO:38 | 8213-8232 | NG_012048.1 |
| *C21ORF45* (+) for | GGTGCTGAGCAGCTGGTGCAAA SEQIDNO:39 | 2936-2957 | NC_000021.8 |
| *C21ORF45* (-) rev | TGCGGGGTCACATTACTCCCCT SEQIDNO:40 | 3093-3114 | NC_000021.8 |
| *CCNE2* (+) for | AAATCCAGGAGTTGCAGTGG SEQIDNO:41 | 13891-13910 | NT_008046.16 |
| *CCNE2* (+) rev | ACTCTCAGGGGCTCCTTCTC SEQIDNO:42 | 13990-14010 | NT_008046.16 |
| *CENPA* (+) for | ACTTCCCAAGGGTCACACAGTCA SEQIDNO:43 | -4444 -4421 | NT_022184.15 |
| *CENPA* (+) rev | TGTGGCAGAATGGGACAGGCG SEQIDNO:44 | -4384 -4363 | NT_022184.15 |
| *CPSF6* (+) for | TGGCAATGAATGCGAGAGGAAGGT SEQIDNO:45 | -626 -605 | NT_029419.12 |
| *CPSF6* (+) rev | CCCCGGGCATTCTGTGAACGG SEQIDNO:46 | -451 -430 | NT_029419.12 |
| *DEPDC1* (+) for | AGGGCCAGGCAGAAAAACCGT SEQIDNO:47 | -442 -421 | NT_032977.9 |
| *DEPDC1* (+) rev | CGGGTTTCCCTGGCGCTGTT SEQIDNO:48 | -277 -257 | NT_032977.9 |
| *DEPDC1* (-) for | GGCTTGGGACGCCTCACGAT SEQIDNO:49 | -2112-2092 | NT_032977.9 |
| *DEPDC1* (-) rev | GCCCATGAACTGCCTAGCTGTGG SEQIDNO:50 | -1932 -1909 | NT_032977.9 |
| *EPB41L4B* (+) for | AGTGCAGCAGTGAGACGGGGA SEQIDNO:51 | -4317-4296 | NT_008470 |
| *EPB41L4B* (+) rev | ACCCCAGAGGCTAGTCCGAAGG SEQIDNO:52 | -4231 -4209 | NT_008470 |
| *FAM64A* (+) for | ATCGGGGCGTCTGGTGGGAA SEQIDNO:53 | 5362-5382 | NT_010718.16 |
| *FAM64A* (+) rev | CGAGGAGGGGAGGCAGGGTC SEQIDNO:54 | 5564-5584 | NT_010718.16 |
| *NCAPH* (+) for | CCCTGCCTGCATCTGTGCCA SEQIDNO:55 | 2477-2497 | NT_022171.15 |

(continued)

| Gene symbol | Sequence | Region of pairing | Reference genome sequence |
|---|---|---|---|
| *NCAPH* (+) *rev* | GCAGGCAAAGAGGTTGCCTTTGT SEQIDNO:56 | 2569-2591 | NT_022171.15 |
| *WDR67* (+) *for* | CACGAGGCTCCCAAAGGGCG SEQIDNO:57 | 79523-79503 | NT_008046 |
| *WDR67* (+) *rev* | AAAATCCGCCCAAGCGGCCA SEQIDNO:58 | 79351-79331 | NT_008046 |

Procedure for detection of the levels of Pin1 in tumour tissue samples

Construction of the tissue microarrays (TMAs)

The tissue microarrays (TMAs) used in this study to ascertain the levels of Pin1 in samples of tumour tissue derive from a historic series of breast carcinoma samples, taken from 212 primary tumours previously untreated and not selected in any other way. The TMAs were created using a specific manual arrayer (Beecher Instruments Inc), with on average six circular sections with a diameter of 0.6 mm per sample. A pathologist expert in the study of the mammary gland (L.J.) labelled for each sample the representative areas of tumour on glass slides stained with haematoxylin and eosin before taking the sections.

Immunolabelling of the TMA

The sections (4 microns in thickness) cut from the TMA blocks were spread out on Superfrost® slides (VWR International Ltd) and dried for 1 hour at 60°C before the paraffin was removed in Histoclear (National Diagnostics). The sections were then rehydrated by immersion in a series of solutions of gradually decreasing alcohol percentage. The sections were incubated in a buffer solution containing 10 mM citric acid at pH 6.0. They were then heated in a microwave oven for 15 min before being immunolabelled using the Dako Autostainer processing system and the Vectastain® ABC kit (Vector Labs), in accordance with the protocol supplied by the manufacturer. Briefly, the sections were blocked for 20 min using normal goat serum, added with 10% (v/v) with a stock solution of avidine (Vector Labs) and then incubated for 1 hour in a solution containing the primary antibody (monoclonal antibody against Pin1 (G-8, sc-46660), dilution 1:250) and added with 10% (v/v) with a stock solution of biotin (Vector Labs), so as to reduce the nonspecific end labelling. The sections were then incubated for 30 minutes in the presence of a biotinylated universal secondary antibody (Vectastain® ABC kit, Vector Labs), and then for another 30 minutes in the presence of the reagent Vectastain® Elite ABC. Liquid diaminobenzidine (DAB) (DAKO) was used as the chromogen, incubating the sections for 5 min. These were then counterstained with a solution of Mayers haematoxylin. During the immunolabelling, between each passage and the next, the slides were washed briefly in Tris-based saline buffer (TBS) at pH 7.6. Each experiment included samples, which could certainly be labelled positively, and negative controls, prepared using TBS buffer in place of the solution containing primary antibody.

Virtual digital microscopy analysis and TMA evaluation

The immunolabelled TMA slides were examined (Scancope XT, Aperio Technologies) using a 40x magnifying objective, and catalogued in the Aperio Spectrum Plus + TMA database (version 9.0.748.1521). The TMAs relating to the cases of breast carcinoma were analysed under blind conditions. The classification of the samples was checked by the same pathologist who conducted the first evaluations, by means of standard analysis by optical microscopy, using a Nikon Eclipse E600 and microscope. Only the circular sections containing tumoral tissue were analysed; criteria excluding sectors from the analysis were: dimensions less than 20% of expectations, the overlapping of the sections, the prevalence of adipose tissue, stroma, or of normal epithelial breast tissue. Thus, 212 tumour samples were sufficient for the evaluation. Only invasive malignant tumours were considered, while tumours *in situ,* the surrounding epithelial and stroma were ignored. The positivity to labelling with the antibodies was evaluated in each section having adequate characteristics and a score using the "Quick Score" method (Detre S. et al., 1995) was assigned. In brief, the percentage of positive cells was calculated and on this basis a score was assigned on the scale from 1 to 6:0 - 4 % = 1; 5 - 19 % = 2; 20 - 39 % = 3; 40 - 59 % = 4; 60 - 79 % = 5 and 80 - 100 % = 6. The mean intensity of the signals coming from labelling of positive cells was evaluated, and the thereto a score from 0 to 3 was assigned: no signal = 0, signal from weak labelling = 1, intermediate signal = 2 and strong signal = 3. The Quick Score was then obtained by multiplying the score relating to the percentage of labelled cells by the score relating to the intensity of the labelling, with a maximum achievable value of 18. Given that, for the majority of the samples, several sections were available for the evaluation, a total Quick Score was obtained that was unique to each tumour, and therefore to each patient. Also the localization type, cytoplasmic or nuclear, of the immunolabelling signal was annotated, and thus each cellular compartment, was separately considered, so obtaining two Quick Score for each patient.

Procedure for analysing the *TP53* status in samples of tumoral tissue

The *TP53* status was analysed by sequencing, using as the starting material the total RNA extracted from samples of frozen breast tumour tissue. The total RNA was extracted using the QIAGEN RNA assay kit. The quality of the nucleic acid recovered was evaluated via the Agilent-bioanalyzer microfluid platform. Samples which, upon analysis, yielded a ratio between the absorbances at 280 nm and 260 nm of less than 1.2 were discarded.

The retrotranscription reaction was conducted starting with 500ng of total RNA using primers specific to p53 so as to amplify all its isoforms.

Primers for analysing the mutational status of *TP53* by sequencing

| Primer | Sequence 5'-3' | Region of pairing | Reference genome sequence |
|--------|----------------|-------------------|---------------------------|
| RT2 | aatgtcagtctgagtcaggcccttctg SEQIDNO:1 | 1383-1357 | NM_000546.4 |
| GIL | tgatgctgtccccggacgatattgaa SEQIDNO:2 | 325-350 | NM_000546.4 |
| MP9ER | tctcccaggacaggcacaaacacg SEQIDNO:3 | 1037-1014 | NM_000546.4 |
| E6E7F | tttgcgtgtggagtatttggat SEQIDNO:4 | 797-818 | NM_000546.4 |
| MP6R | ttggcaaaacatcttgttgagggc SEQIDNO:5 | 605-582 | NM_000546.4 |

Methods for determining the presence of the *molecular signature* via gRT-PCR

From the expression matrix for the dataset of Desmedt et al., 2007 (GSE7390), the expression values were extracted for the genes showing the lowest variation (standard deviation less than 0.4) between the samples; among these genes, three were identified, having absolute expression values included within the absolute variation of expression of the 10 genes. A score for the *molecular signature* was then constructed, adding the relative gene score evaluating for each of the genes the relative expression with respect to one of the reference genes, as in the table below:

| "Molecular signature" | "Reference genes" |
|-----------------------|-------------------|
| FAM64A | ADCY3 |
| CENPA | ADCY3 |
| C21 orf45 | ADCY3 |
| NCAPH | ADYC3 |
| BUB1 | ERCC6 |
| CCN2 | ERCC6 |
| WDR67 | ERCC6 |
| DEPDC1 | ERCC6 |
| CPSF6 | TUBGCP2 |
| EPB41L4B | TUBGCP2 |

In particular, the gene score of the *molecular signature* is calculated on the basis of the following formula:

$$Sg= \begin{cases} Log(Exp_i, base=2)-log(Exp_r, base=2)< -0.5 \implies Sg=0 \\ \\ Log(Exp_i, base=2)-log(Exp_r, base=2) \geq -0.5 \\ Log(Exp_i, base=2)-log(Exp_r, base=2) \leq 0.5 \end{cases} \implies Sg=1 \\ Log(Exp_i, base=2)-log(Exp_r, base=2)> 0.5 \implies Sg=2$$

where:

a) if a gene $i$ of the *molecular signature* has an expression value $Exp_i$ less than 0.5 on the base 2 logarithmic scale by comparison with the corresponding expression value $Exp_r$ of its reference gene $r$ (equivalent to saying that there exists a difference of 0.5 RT-PCR cycles), this gene is attributed a gene score of zero.

b) if a gene $i$ of the *molecular signature* has an expression value $Exp_i$ higher than 0.5 on the base 2 logarithmic scale by comparison with the corresponding expression value $Exp_r$ of its reference gene $r$ (equivalent to saying that there exists a difference of 0.5 RT-PCR cycles), this gene is attributed a gene score of two.

c) if a gene $i$ of the *molecular signature* has an expression value $Exp_i$ intermediate to the two values of cases a) and b) by comparison with the corresponding expression value $Exp_r$ of its reference gene $r$, this gene is attributed a gene score of one.

The sum of the genes scores (Sg) determines the value of the score for the *molecular signature* of the 10 genes ($S_f$):

$$S_f = \sum S_g$$

According to the value of $S_f$, it is possible to evaluate the prognosis of the patient on the basis of the following criteria:

- a score value of the *molecular signature* of the ten genes ($S_f$) lower than or equal to 5 is associated with a favourable prognosis (80% without metastases at ten years);
- a score value of the *molecular signature* of the ten genes ($S_f$) lower than or equal to 9 and above 5 is associated with a less favourable prognosis (60% without metastases at ten years);
- a value above 9 is associated with a less favourable prognosis (50% without metastases at ten years).

Analysis of the survival data on the basis of TMA

The survival of the patient was calculated using as time range the time from the date on which treatment was started to the date of death, or, if the patient was still alive, the date on which the assessment was carried out. The survival curves were constructed using the Kaplan-Meier method. The differences between the various tumour characteristics are estimated by means of the log-rank test. The chemotherapeutic treatment was carried out according to a classical protocol based on anthracycline.

Multivariate analysis according to the Cox proportional hazards model

To calculate the death risk in the 212 tumour cases included in the TMAs, an analysis using the Cox proportional hazards regression model was conducted. Parameters examined were the relationship existing between patient survival, the presence of missense in *TP53* and the levels of Pin1 and the status of a series of other predictive parameters commonly used in clinical practice, including the tumour size, the presence or absence of receptors for oestrogen (ER status) or progesterone (PGR status), the lymph node status, the expression levels of HER2, the existence of lymph-node infiltration, and the tumour grade. The Cox proportional hazards regression model was applied in the first instance to the clinical variables available. The variables related to the presence of missense mutations in *TP53,* and to the level of Pin1 were then added. The clinical parameters were considered in the following manner:

- Tumour size: continuous variable;
- Tumour grade: grade 1, grade 2, grade 3;
- Lymph-node infiltration: negative if less than or equal to 4 (0), positive if > 4 (1);
- ER status: negative if less than or equal to 4 (1), positive if > 4 (0);
- PGR status: negative if less than or equal to 4 (1), positive if > 4 (0);
- HER2 status: positive or negative to staining (0, 1);
- Pin1 status: Pin1 nuclear staining positive if > 5 (1), negative if less than or equal to 5 (0).

**Results**

Pin1 increases tumoral transformation in a mutant p53 *"knockin"* mouse model

In order to understand the type of influence exerted by Pin1 on mutant p53 during tumorigenesis, mice *"knockin"* for mutant p53 - bearing a *"knockin"* allele coding for the protein p53R172H ($p53^{M/+}$) - (Lang G.A. et al., 2004) were crossed with *Pin1*$^{+/-}$ mice (Atchinson F.W. et al., 2003). Two cohorts of mice derived from these crosses, composed of animals with the wild-type or knock-out genotype for Pin1 and bearing one or two alleles knockin for mutant p53 ($p53^{M/+}$ *Pin1*$^{+/+}$, $p53^{M/+}$ *Pin1*$^{-/-}$ and $p53^{M/M}$ *Pin1*$^{+/+}$, $p53^{M/M}$ *Pin1*$^{-/-}$) were generated and analysed. The survival data relating to the $p53^{M/M}$ *Pin1*$^{+/+}$ and $p53^{M/+}$ *Pin1*$^{+/+}$ were consistent with previously published studies (Lang G.A. et al., 2004). In both cohorts to which the mice lacking Pin1 belonged, the median survival in the absence of tumours was higher (Fig. 1A and 1B). In the cohort of $p53^{M/M}$ mice, the absence of Pin1 caused only the frequency variation of some tumour types (Table 7).

Table 7: Spectrum of tumours in the $p53^{M/M}$ mouse cohort

| | p53$^{M/M}$ Pin1$^{+/+}$ (n=27) | p53$^{M/M}$ Pin1$^{-/-}$ (n=22) |
|---|---|---|
| **Haematopoietic lymphoid neoplasias** | **61 % (20/33)** | **42 % (11/26)** |
| Thymic lymphoma | (16) | (7) |
| Multicentric lymphoma | (2) | (2) |
| Splenic follicular lymphoma | (2) | (2) |
| | | |
| **Haematopoietic non-lymphoil neoplasias** | **9 % (3/33)** | **23 % (6/26)** |
| Histiocytic sarcoma | (2) | (6) |
| Myeloid leukaemia | (1) | (-) |
| **Carcinomas** | **3 % (1/33)** | **4 % (1/26)** |
| Carcinoma of the prostate | (1) | (1) |
| | | |
| **Sarcomas** | **27 % (9/33)** | **23 % (6/26)** |
| Osteosarcoma | (0) | (1) |
| Haemangiosarcoma | (6) | (4) |
| Undifferentiated sarcoma of the soft tissues | (3) | (1) |
| | | |
| **Other** | **0 % (0/33)** | **8 % (2/26)** |
| Primitive neuroectodermal tumour | (-) | (1) |
| Nephroblastoma | (-) | (1) |
| **Total Tumours** | **33** | **26** |

With regard to the cohort of $p53^{M/+}$ *Pin1*$^{-/-}$ mice as compared to $p53^{M/+}$ *Pin1*$^{+/+}$ mice, however, a markedly reduction in the frequency of tumours, a reduced number of lymphomas, and the complete absence of carcinomas were observed (Fig. 1C, Table 8).

Table 8: Spectrum of tumours in the $p53^{M/+}$ e $p53^{+/-}$ mouse cohorts

| | p53$^{M/+}$ Pin1$^{+/+}$ | p53$^{M/+}$ Pin1$^{-/-}$ | p53$^{+/-}$ Pin1$^{+/+}$ | p53$^{+/-}$ Pin1$^{-/-}$ |
|---|---|---|---|---|
| **Haematopoietic and lymphoid neoplasias** | **18% (4/22)** | **12.5 % (1/8)** | **15% (3/20)** | **20% (4/20)** |
| Thymic lymphoma | (1) | (1) | (1) | (-) |
| Multicentric lymphoma | (3) | (-) | (2) | (4) |
| | | | | |
| **Haematopoietic and non-lymphoid neoplasias** | **14% (3/22)** | **25 % (2/8)** | **13% (3/20)** | **25% (5/20)** |
| Histiocyticsarcoma | (2) | (5) | (3) | (5) |
| Myeloid leukaemia | (1) | (-) | (-) | (-) |
| **Carcinomas** | **27% (6/22)** | **0 % (0/8)** | **0 % (0/20)** | **5 % (1/20)** |
| Cholangiocarcinoma | (1) | (-) | (-) | (-) |
| Hepatocarcinoma | (1) | (-) | (-) | (-) |
| Intestinal adenocarcinoma | (1) | (-) | (-) | (-) |
| Undifferentiated prostatic carcinoma | (1) | (-) | (-) | (-) |
| Undifferentiated mammary carcinoma | (1) | (-) | (-) | (-) |
| Urothelial carcinoma of the renal pelvis | (1) | (-) | (-) | (-) |
| Squamous-cell gastric carcinoma | (-) | (-) | (-) | (1) |
| **Sarcomas** | **36% (8/22)** | **62.5 % (5/8)** | **70% (14/20)** | **50% (10/20)** |
| Osteosarcoma | (3) | (2) | (6) | (3) |
| Haemangiosarcoma | (2) | (1) | (-) | (1) |
| Undifferentiated sarcoma of the soft tissues | (3) | (2) | (7) | (6) |
| Rhabdosarcoma | (-) | (-) | (1) | (-) |
| | | | | |
| **Other** | **5 % (1/22)** | **0 % (0/8)** | **0 % (0/20)** | **0 % (0/20)** |
| Pituitary adenoma | (1) | (-) | (-) | (-) |
| **Total tumours** | **22** | **8** | **20** | **20** |

Mice of the genotypes $p53^{+/-}$ Pin1$^{+/+}$ and $p53^{+/-}$ Pin1$^{-/-}$ were also taken into consideration, for which were recorded no differences in tumour-free survival, in the frequency or spectrum of the tumours (Fig.s 2A and 2B, Table 8).

In mice, therefore, the absence of Pin1 is associated with the reduction in tumorigenesis phenomena only in one context in which mutant p53 is expressed. From these data, it can therefore be deduced that Pin1 and mutant p53 co-operate during the events which lead to tumoral transformation.

Regarding the mechanisms underlying this genetic interaction between Pin1 and mutant p53 it has emerged that, in samples of tumour tissue from $p53^{M/+}$ Pin1$^{+/+}$ mice, Pin1 co-immunoprecipitated with p53. Given that the enzyme specifically recognises the phosphorylated S/T-P motifs on its own substrates, these observations lead one to suppose that Pin1 may bind mutant p53, regulating its function in response to the phosphorylation signals produced by oncogenic stress.

The effect of Pin1 on the ability of mutant p53 to favour in primary cells the tumoral transformation induced by Ras (Lang G.A. et al., 2004) was evaluated on embryo fibroblasts (MEF) obtained from $p53^{M/M}$ Pin1$^{+/+}$, $p53^{M/M}$ Pin1$^{-/-}$, $p53^{-/-}$ Pin1$^{+/+}$ and $p53^{-/-}$ Pin1$^{-/-}$ mice. The effect was analysed in assays directed at ascertaining their growth capacity independently of anchorage to the substrates and their tumorigenesis potential *in vivo* following introduction of the H-Ras$^{V12}$ cells by means of retroviral vectors. The $p53^{M/M}$ Pin1$^{+/+}$ cells proved capable of giving rise to two-fold more the number of colonies in soft agar (Fig. 1D, left), and, once injected subcutaneously into immunocompromised mice, they were capable of developing tumours double in size compared with those originated by the *p53$^{M/M}$ Pin1$^{-/-}$* and *p53$^{-/-}$* cells (Fig. 1D, right and Fig. 2C).

In MEF *p53$^{M/M}$ Pin1$^{+/+}$* cells transduced with H-Ras$^{V12}$, in agreement with the results obtained in previous studies, it was observed that overexpression of the protein H-Ras$^{V12}$ caused an increase in the levels of mutant p53 and of Pin1 (Ryo A. et al., 2002). Moreover, the immunoblotting experiments, in which specific antibodies were used, which recognise phosphorylated S/T-P amino acid residues, have demonstrated that the overexpression of H-Ras$^{V12}$ lead to an increase in the levels of mutant p53 phosphorylation, at the level of the amino acid residues constituting the motifs for recognition and binding by Pin1 (Fig. 1E, top). In accordance with what has been observed, the interaction of mutant p53 with Pin1 was potentiated (Fig. 1E, bottom).

Overall, these results suggest that Pin1 is necessary for the ability of mutant p53 to promote cell transformation and tumoral progression, reveal for the enzyme, a crucial role as an oncogenic signalling transducer, which leads to activation of mutant p53 gain of function.

Pin1 potentiates the effects of mutant p53 in favour of cell migration and invasion of human tumour cell lines

Also in human tumour cells of the lines MDA-MB-231 and SK-BR-3, derived from breast tumours, it has been observed that the enzyme binds the protein endogenous mutant p53. To establish whether Pin1 is able, in this context, to promote the oncogenic functions of mutant p53, the role of Pin1 in the induction of cell migration and invasion was evaluated, these being the critical aspects of the metastatic phenotype, which is manifested following the action of mutant p53 (Adorno M. et al., 2009; Muller P. et al., 2009). Knocking down by means of RNAi the expression levels of either mutant p53 or Pin1, a significant reduction in the ability of MDA-MB-231 to migrate and to be invasive, has been obtained (Fig. 3A). The effect exerted by Pin1 on migration was specific and was necessarily expressed in the presence of its own enzymatic activity. Indeed, by re-introducing Pin1 into cells by means of a construct resistant to silencing (Pin1 r), it was possible to recover the ability of said cells to migrate. However, this did not occur if the element supplied was a construct coding for a mutant of Pin1 (Pin1 rS67E) that was catalytically inactive (Fig. 3B). The influence of the enzyme in promoting the capacity to invade and to migrate was then evaluated *in vivo* by injecting intravenously in immunocompromised mice Pin1-depleted or control MDA-MB-231 cells. The enzyme-depleted cells showed a markedly reduced capacity to invade the lungs (Fig. 3C).

The next step was therefore to investigate whether Pin1 is capable of producing the observed effects epistatically, by modulating the mutant p53 functions. As shown in Fig. 3D, overexpression of Pin1 in MDA-MB-231 cells caused a significant increase in the migration and invasive capacity of these cells, but if the levels of mutant p53 are simultaneously depleted, the observed effects are almost completely abolished.

Overall, these results indicate that the binding of Pin1 to mutant p53 triggers the action of mutant p53 in favour of cell migration.

The Pin1/mutant p53 axis activates a specific transcription programme, which favours the aggressive tumour phenotype

In the attempt to understand how Pin1 and mutant p53 are able globally to alter gene expression, the transcriptional profile of MDA-MB-231 cells was analysed after depletion of either Pin1 or mutant p53. In both cases, an effect on the transcription of genes belonging to the same functional categories was observed, suggesting that Pin1 and mutant p53 are involved in the regulation of similar cellular processes, including proliferation, motility, dynamic organisation of the cytoskeleton, metabolism and signalling transduction. In particular, 386 genes were identified which, following depletion of either Pin1 or mutant p53 levels, are up-regulated, and 303 which, on the other hand, are down-regulated under the same conditions. On the basis of this result, it may be suppose that one group of genes is regulated by the concerted action of these two proteins (Fig. 4A).

The effect caused by depletion of either Pin1 or mutant p53 on the expression of the selected genes has been confirmed by qRT-PCR (Fig. 5A).

The transcription programme induced by the combined action of mutant p53 and Pin1 includes genes which play a relevant role in determining tumour aggressiveness, and may therefore influence breast cancer prognosis. To test this hypothesis, an analysis was conducted on primary tumours in the search for a correlation between the expression of genes induced by Pin1 and mutant p53 (that is, those genes down-regulated in the transcriptional profiling experiments), and the disease prognosis , by using four independent data sets relating to a total of 800 cases of breast cancer (Desmedt C. et al., 2007; Miller L.D. et al., 2005; Pawitan Y. et al., 2005; Sotiriou C. et al., 2006). From among the 303 down-regulated genes, those selected for the analysis were the subgroup (consisting of 31 genes) of genes most repressed under both silencing conditions. The Desmedt dataset (Desmedt C. et al., 2007) was used as a "training set" for construction of a system of gene classification based on a correlation with the clinical data (see Table 2).

From the classification of genes based on the association with clinical data, the genes having a score of more than 3 were selected, thus obtaining a *molecular signature* associated with Pin1/mutant p53, consisting of the 10 genes listed in Table 2 mentioned above.

The expression of these genes correlate significantly with the clinical outcome of the disease: in patients who expressed these genes at high levels, the time interval between diagnosis of the primary tumour and that of metastases in other regions of the body *(Time to Distant Metastasis,* TDM) was shorter and survival was reduced *(Overall Survival,* OS) (Fig. 4B e 6A-D). The expression of these 10 genes also has prognostic value considering exclusively the patients with mammary tumour positive to the receptor for oestrogen (Fig. 6E and 6F). Similar results were obtained by analysing the expression of a group of seven genes consisting of: *C21orf45, CPSF6, DEPDC1, EPB41L4B, FAM64A, NCAPH* and *WDR67* (data not shown).

The subsequent step was to test whether these 10 genes were regulated by the concerted action of Pin1 and mutant p53. As shown in Fig. 4C, in MDA-MB-231 cells depleted of the endogenous mutant p53 protein, the introduction of a construct coding for p53K280, but not of one coding for expression of the protein p53K280-4M (a modified form of endogenous mutant p53 (p53K280), unable to bind Pin1, in which the amino acid alanine substitutes the residues of serine or of threonine present at the level of the four S/T-P sites (S33, S46, T81 and S315) of phosphorylation and of binding to Pin1), induced the expression of all these genes. However, the still more important result is that, when Pin1 was ectopically expressed together with p53K280, the expression of these genes was strongly induced. If, on the other hand, Pin1 was over expressed alone or together with p53K280-4M, no effect on said genes was observed (Fig. 4C).

To analyse in detail the mechanism underlying the transcriptional activation mediated by the concerted action of Pin1 and mutant p53, chromatin immunoprecipitation (ChIP) experiments were conducted in MDA-MB-231 cells. As shown in Fig. 4D, mutant p53 is specifically recruited on the promoters of all the genes belonging to the molecular signature associated to Pin1/mutant p53. Following the silencing of Pin1, however, its recruitment was reduced approximately two-fold. On the other hand, also Pin1 is present on the same promoters, but in a mutant p53 dependent manner (Fig. 4E).

Overall, the data indicate that these genes are direct targets for the action of transcriptional induction exerted by the Pin1/mutant p53 axis, and that Pin1 could be required for the proper interaction of mutant p53 with various functional sites present on chromatin. In this regard, a more in-depth analysis of these promoter regions has revealed the presence of sites of recognition and binding for many transcription factors known for their ability to interact with mutant p53. These include Ets-1, NF-Y, Sp1 and VDR (Brosh R. and Rotter V., 2009) (data not shown).

The majority of these genes proved to be regulated by Pin1 and mutant p53 even in MDA-MB-468 cells which, as the endogenous mutant form, express the protein p53H273 (Fig. 5B). This gives rise to the supposition that Pin1 can influence the transcriptional activity of other mutant p53 proteins.

To attempt to understand the functional role of these 10 genes as mediators of Pin1/mutant p53-dependent cell migration, we analysed what happens to the MDA-MB-231 cells following their silencing, and identified six genes, whose silencing had the effect of reducing the migration ability of these cells. Depletion of the levels of *DEPDC1* caused the more marked effect, with an impact also on the invasiveness of these cells.

Overall, these results indicate that Pin1 acts in concert with mutant p53 in reprogramming gene expression of cancer cells, by activating a specific transcriptional programme in which a number of genes, that are important for the cell migration and invasion, takes part. These genes had not been identified up to now as direct targets of the transcriptional action of mutant p53.

Overexpression of Pin1 and the presence of mutations in p53, influence the clinical outcome of breast tumours

The next step in the analysis was to evaluate in breast tumour, the type of association existing between the expression of Pin1 and the mutational status of p53, and the clinical outcome of the disease. Quantitative immunohistochemical assays were conducted for the purpose of evaluating the expression of Pin1 and of p53 in primary mammary carcinoma. The p53 status was analysed by means of direct sequencing, and only those tumours proving to bear the wild-type or missense mutations alleles were included in this study, for a total of 212 samples. Pin1 was shown to be over expressed in 144 out of 212 cases (68%), whereas in 46 out of 212 (22%), missense mutations in *TP53* were found (Table 9).

Table 9: TMA dataset

|  | p53 wild type | mutant p53 | Total |
|---|---|---|---|
| **Elevated Pin1** | 111 | 32 | 143 |
| **Low Pin1** | 55 | 14 | 69 |
| **Total** | 166 | 46 | 212 |

No association emerged between the survival probability and the overexpression of Pin1 (Fig. 8A). However, in agreement with observations of other studies (Langerød A. et al., 2007; Olivier M. et al., 2006) a reduction in survival was observed in the cases in which missense mutations in p53 were present (Fig. 8B). It should be emphasised that, when the probability of survival was evaluated as a function of the combination of the expression levels of Pin1 and the presence of missense mutations in p53, it emerged that the survival probability was significantly reduced in those cancer cases which expressed elevated levels of Pin1 (Pin1 high) and mutant p53, compared to those in which the expression of mutant p53 was associated with the presence of low levels of Pin1 (Pin1 low) or to those in which the protein p53 was "wild type" (Fig. 7A). The multivariate analysis, obtained using the Cox proportional hazards model, furthermore demonstrated that the presence of an overexpression of Pin1 combined with the presence of a mutation in p53 constituted an independent predictive factor of the clinical outcome of the disease (Fig. 7B).

Evidence of the fact that the prognostic value of the presence of mutation in p53 depends on the Pin1 expression levels is further reinforced by the observation that the correlation between the presence of mutation in p53 and the decrease in the survival probability is present only in cases in which expression of Pin1 is high (Fig. 7C and 7D). It has been observed that, in patients undergoing anthracycline-based adjuvant chemotherapy, the expression of Pin1 and the presence of missense mutations in p53 proved to be associated with an unfavourable outcome of the disease. In this subgroup of patients also, the combination between overexpression of Pin1 and the presence of mutation in p53 was revealed to be an independent prognostic factor (Fig. 8C).

Overall, therefore, the data produced by the inventors indicate that, in cancer cells, the combined action of Pin1 and mutant p53 has a deep impact on the programme of gene expression by directly activating a transcriptional programme, which promotes the aggressive tumoral phenotype (Fig. 9).

**References**

- Adorno, M., Cordenonsi, M., Montagner, M., Dupont, S., Wong, C., Hann, B., Solari, A., Bobisse, S., Rondina, M. B., Guzzardo, V. et al. (2009). A Mutant-p53/Smad Complex Opposes p63 to Empower TGFb-Induced Metastasis. Cell 137, 87-98.
- Atchison, F. W., Capel, B., and Means, A. R. (2003). Pin1 regulates the timing of mammalian primordial germ cell proliferation. Development 130, 3579-3586.
- Bao, L., Kimzey, A., Sauter, G., Sowadski, J. M., Lu, K. P., and Wang, D. G. (2004). Prevalent overexpression of prolyl isomerase Pin1 in human cancers. Am. J. Pathol. 164, 1727-1737.
- Brosh, R. and Rotter, V. (2009). When mutants gain new powers: news from the mutant p53 field. Nat. Rev. Cancer 9, 701-713.
- Caulin, C., Nguyen, T., Lang, G. A., Goepfert, T. M., Brinkley, B. R., Cai, W., Lozano, G., and Roop, D. R. (2007). An inducible mouse model for skin cancer reveals distinct roles for gain- and loss-of-function p53 mutations. J. Clin. Invest. 117, 1893-1901.
- Coughlin, S.S., Ekwueme, D.U. (2009). Breast cancer as a global health concern. Cancer Epidemiol. 33, 315-8.
- Desmedt, C., Piette, F., Loi, S., Wang, Y., Lallemand, F., Haibe-Kains, B., Viale, G., De Lorenzi, M., Zhang, Y., Sagatchian D'Assignies, M. et al. (2007). Strong Time Dependence of the 76-Gene Prognostic Signature for Node-Negative Breast Cancer Patients in the TRANSBIG Multicenter IndependentValidation Series. Clin. Cancer Res. 13, 3207-3214.
- Detre, S., Saccani Jotti, G., and Dowsett, M. (1995). "quickscore" method for immunohistochemical semiquantitation: validation for oestrogen receptor in breast carcinomas. J. Clin. Pathol. 48, 876-878.
- Drost, J., Mantovani, F., Tocco, F., Elkon, R., Comel, A., Holstege, H., Kerkhoven, R., Jonkers, J., Voorhoeve, P.M., Agami, R., Del Sal, G. (2010). BRD7 is a candidate tumour suppressor gene required for p53 function. Nat Cell Biol. 12, 380-9.
- Efeyan, A. Murga, M., Martinez-Pastor, B., Ortega-Molina, A., Soria, R., Collado, M., Fernandez-Capetillo, O. and Serrano M. (2009). Limited Role of Murine ATM in Oncogene-Induced Senescence and p53-Dependent Tumor Suppression. PLoS ONE. 4 (5): e5475.
- Gentleman, R. C., Carey, V. J., Bates, D. M., Bolstad, B., Dettling, M., Dudoit, S., Ellis, B., and Gaultier, L. (2004). Bioconductor: open software development for computational biology and bioinformatics. Genome Biol. 5, R80.
- Guo, A. M., Coffill, C. R., and Lane, D. (2011). The role of mutant p53 in human cancer. J of Pathology. 223, 116-126.
- Irizarry, R. A., Hobbs, B., Collin, F., Beazer-Barclay, Y. D., Antonellis, K. J., Scherf, U., and Speed, T. P. (2003). Exploration, normalization, and summaries of high density oligonucleotide array probe level data. Biostatistics 4, 249-264.
- Jemal, A., Siegel, R., Ward, E., Hao, Y., Xu, J., and Thun, M.J. (2009). Cancer statistics. CA Cancer J Clin. 59, 225-49.
- Jemal, A., Bray, F., Center, M.M., Ferlay, J., Ward, E., and Forman, D. (2011). Global cancer statistics. CA Cancer J Clin. 61, 69-90.

- La Vecchia, C., Bosetti, C., Lucchini, F., Bertuccio, P., Negri, E., Boyle, P., and Levi, F. (2010). Cancer mortality in Europe, 2000-2004, and an overview of trends since 1975. Ann Oncol. 21, 1323-60.

- Lang, G. A., Iwakuma, T., Suh, Y., Liu, G., Rao, A., Parant, J. M., Valentin-Vega, Y. A., Terzian, T., Caldwell, L. C., Strong, L. C. et al. (2004). Gain of Function of a p53 Hot Spot Mutation in a Mouse Model of Li-Fraumeni Syndrome. Cell 119, 861-872.

- Langerød, A., Zhao, H., Borgan, O., Nesland, J. M., Bukholm, I. R. K., Ikdahl, T., Kåresen, R., Børresen-Dale, A. L., and Jeffrey, S. S. (2007). TP53 mutation status and gene expression profiles are powerful prognostic markers of breast cancer. Breast Cancer Res. 9, R30

- Lu, K. P. and Zhou, X. Z. (2007). The prolyl isomerase PIN1: a pivotal new twist in phosphorylation signalling and disease. Nat. Rev. Mol. Cell Biol. 8, 904-916.

- Malkin, D., Li, F. P., Strong, L. C., Fraumeni, J. F. Jr., Nelson, C. E., Kim, D. H., Kassel, J., Gryka, M. A., Bischoff, F. Z., Tainsky, M. A. et al. (1990). Germ line p53 mutations in a familial syndrome of breast cancer, sarcomas, and other neoplasms. Science 250, 1233-1238.

- Mantovani, F., Tocco, F., Girardini, J. E., Smith, P., Gasco, M., Lu, X., Crook, T., and Del Sal, G. (2007). The prolyl isomerase Pin1 orchestrates p53 acetylation and dissociation from the apoptosis inhibitor iASPP. Nat. Struct. Mol. Biol. 14, 912-920.

- Miller, L. D., Smeds, J., George, J., Vega, V. B., Vergara, L., Ploner, A., Pawitan, Y., Hall, P., Klaar, S., Liu, E. T. et al. (2005). An expression signature for p53 status in human breast cancer predicts mutation status, transcriptional effects, and patient survival. Proc. Natl. Acad. Sci. USA. 102, 13550-13555.

- Muller, P., Caswell, P. T., Doyle, B., Iwanicki, M. P., Tan, E. H., Karim, S., Lukashchuk, N., Gillespie, D. A., Ludwig, R. L., Gosselin, P. et al. (2009). Mutant p53 Drives Invasion by Promoting Integrin Recycling. Cell 139, 1327-1341.

- Olivier, M., Langerød, A., Carrieri, P., Bergh, J., Klaar, S., Eyfjord, J., Theillet, C., Rodriguez, C., Lidereau, R., Bieche, I. et al. (2006). The Clinical Value of Somatic TP53 GeneMutations in 1,794 Patients with Breast Cancer. Clin. Cancer. Res. 12, 1157-1167.

- Pawitan, Y., Bjöhle, J., Amler, L., Borg, A. L., Egyhazi, S., Hall, P., Han, X., Holmberg, L., Huang, F., Klaar, S. et al. (2005). Gene expression profiling spares early breast cancer patients from adjuvant therapy: derived and validated in two population-based cohorts. Breast Cancer Res. 7, R953-964.

- Prat, A., Perou, C.M. (2011). Deconstructing the molecular portraits of breast cancer. Mol Oncol. 5, 5-23.

- Rustighi, A., Tiberi, L., Soldano, A., Napoli, M., Nuciforo, P., Rosato, A., Kaplan, F., Capobianco, A., Pece, S., Di Fiore, P. P. et al. (2009). The prolyl-isomerase Pin1 is a Notch1 target that enhances Notch1 activation in cancer. Nat. Cell Biol. 11, 133-142.

- Ryo, A., Liou, Y. C., Wulf, G., Nakamura, M., Lee, S. W., and Lu, K. P. (2002). PIN1 Is an E2F Target Gene Essential for Neu/Ras-Induced Transformation of Mammary Epithelial Cells. Mol. Cell. Biol. 22, 5281-5295.

- Smyth, G. K. (2004). Linear models and empirical bayes methods for assessing diferential expression in microarray experiments. Stat Appl Genet Mol Biol 3, Article 3.

- Song, H., Hollstein, M., and Xu, Y. (2007). p53 gain-of-function cancer mutants induce genetic instability by inactivating ATM. Nature Cell Biology 9, 573-580.

- Sotiriou, C., Wirapati, P., Loi, S., Harris, A., Fox, S., Smeds, J., Nordgren, H., Farmer, P., Praz, V., Haibe-Kains, B. et al. (2006). Gene expression profiling in breast cancer: understanding the molecular basis of histologic grade to improve prognosis. J. Natl. Cancer Inst. 98, 262-272.

- Soussi, T. and Beroud, C. (2001). Assessing TP53 status in human tumours to evaluate clinical outcome. Nature Reviews on Cancer 1, 233-240.

- Soussi, T. and Wiman, K. G. (2007). Shaping Genetic Alterations in Human Cancer: The p53 Mutation Paradigm. Cancer Cell 12, 303-312.

- Terzian, T., Suh, Y., Iwakuma, T., Post, S. M., Neumann, M., Lang, G. A., Van Pelt, C. S., and Lozano, G. (2008). The inherent instability of mutant p53 is alleviated by Mdm2 or p16INK4a loss. Genes & Dev. 22, 1337-1344.

- UKCCCR (1989). UKCCCR guidelines for the welfare of animals in experimental neoplasia. Cancer Metastasis Rev. 8, 82-88.

- van't Veer, L. J., Dai, H., van de Vijver, M. J., He, Y. D., Hart, A. A., Mao, M., Peterse, H. L., van der Kooy, K., Marton, M. J., Witeveen, A. T. et al. (2002). Gene expression profiling predicts clinical outcome of breast cancer. Nature 415, 530-536.

- Wulf, G., Garg, P., Liou, Y. C., Iglehart, D., and Lu, K. P. (2004). Modeling breast cancer in vivo and ex vivo reveals an essential role of Pin1 in tumorigenesis. EMBO J. 23, 3397-3407.

- Yeh, E. and Means, A. R. (2007). PIN1, the cell cycle and cancer. Nat. Rev. Cancer 7, 381-388.

- Zacchi, P., Gostissa, M., Uchida, T., Salvagno, C., Avolio, F., Voliniak, S., Ronai, Z., Blandino, G., Schneider, C., and Del Sal, G. (2002). The prolyl isomerase Pin1 reveals a mechanism to control p53 functions after genotoxic insults. Nature 419, 853-857.

- Zheng, H., You, H., Zhou, X. Z., Murray, S. A., Uchida, T., Wulf, G., Tang, X., Lu, K. P., and Xiao, Z.-X. (2002). The Prolyl isomerase Pin1 is a novel regulator of p53 in genotoxic response. Nature 419, 849-853.

## Claims

1. A method for breast cancer prognosis comprising the detection in a biopsy sample of a *molecular signature* consisting of the expression of a panel of genes formed by: *DEPDC1, CPSF6, C21orf45, FAM64A, EPB41L4B, NCAPH, WDR67, CENPA, CCNE2, BUB1* or of the proteins expressed thereby, wherein the overexpression of the *molecular signature* genes is correlated to a poor prognosis.

2. The method for breast cancer prognosis based on the detection of a *molecular signature* consisting of the expression of a panel of genes according to claim 1 comprising at least the steps of:

   - acquiring the signal of the expression of the genes forming the *molecular signature DEPDC1, CPSF6, C21orf45, FAM64A, EPB41L4B, NCAPH, WDR67, CENPA, CCNE2, BUB1;*
   - acquiring the signal of the expression of a panel of reference genes consisting of at least *ERCC6, ADCY3, TUBGCP2;*
   - classifying the sample in the group of good, intermediate or poor prognosis on the basis of a score obtained by the sum of the gene score ($S_g$) of each gene calculated by evaluating the relative expression of each *molecular signature* gene with the expression of the reference gene

$$S_f = \sum S_g$$

   where to a value of $S_f$ lower than 5 is associated to a good prognosis, to a value of $S_f$ lower than 9 and higher than 5 is associated to an intermediate prognosis and to a value of $S_f$ higher than 9 is associated to a poor prognosis.

3. The method for breast cancer prognosis according to claim 2, wherein the gene score ($S_g$) is calculated on the basis of the formula

$$S_g = \begin{cases} \text{Log}(Exp_i, base=2) - \text{log}(Exp_r, base=2) < -0.5 \implies S_g = 0 \\[2ex] \begin{aligned} \text{Log}(Exp_i, base=2) - \text{log}(Exp_r, base=2) &\geq -0.5 \\ \text{Log}(Exp_i, base=2) - \text{log}(Exp_r, base=2) &\leq 0.5 \end{aligned} \implies S_g = 1 \\[2ex] \text{Log}(Exp_i, base=2) - \text{log}(Exp_r, base=2) > 0.5 \implies S_g = 2 \end{cases}$$

where:

- $S_g$ is equal to 0 if a gene *l* of the *molecular signature* has an expression value $Exp_i$ lower than -0.5 on the base 2 logarithmic scale by comparison with the corresponding expression value $Exp_r$ of its reference gene *r*,
- $S_g$ is equal to 2 if a gene *l* of the *molecular signature* has an expression value $Exp_i$ higher than 0.5 on the base 2 logarithmic scale by comparison with the corresponding expression value $Exp_r$ of its reference gene *r*; and
- $S_g$ is equal to 1 if a gene *l* of the *molecular signature* has an expression value $Exp_i$ intermediate between lower than -0.5 and higher than 0.5 on the base 2 logarithmic scale by comparison with the corresponding expression value Expr of its reference gene *r*.

4. The method for breast cancer prognosis according to claim 2, wherein the reference gene for the genes *FAM64A, CENPA, C21orf45, NCAPH,* is the gene *ADCY3,* for the genes *BUB1, CCN2, WDR67* is the gene *ERCC6* and for the genes *CPSF6* and *EPB41L4B* is the gene *TUBGCP2.*

5. The method for breast cancer prognosis according to claims 2-4, wherein the expression of the *molecular signature* genes and reference genes is determined by molecular techniques selected from PCR, microarray and sequencing.

6. The method for breast cancer prognosis according to claim 5, wherein for the expression determination of the *molecular signature* genes and reference genes the oligonucleotides of sequence from SEQ ID NO:6 to SEQ ID NO:31 are used.

7. The method for breast cancer prognosis based on the detection in a biopsy sample of the detection of a *molecular signature* consisting of the expression of a panel of genes according to claim 1, wherein the gene expression is determined by the expression of proteins thereby comprising at least the steps of:

   - acquiring an immunohistochemical signal of the expression of the protein expressed by the genes;
   - measuring thereof and assigning to the sample a score by means of the Quick score method;
   - classifying the sample in the group of good or poor prognosis on the basis of a score for protein expression lower or higher than 5.

8. The method for breast cancer prognosis according to claim 7, wherein the Quick score is calculated on the basis of the steps of:

   - evaluating the positivity to labeling with antibodies by calculating the percentage of the positive cells and assigning a score from 1 to 6: 0-4% =1; 5-19%=2; 20-29%=3; 40-59%=4; 60-79%=5 and 80-100%= 6;
   - detecting the signal deriving from labeling and evaluating the average intensity with assignment of a score from 0 to 3: no signal=0; weak labelling signal = 1; medium labeling signal = 2; strong labeling signal = 3; and
   - multiplying the score on the positive cell percentage and score on the signal intensity.

9. The method for breast cancer prognosis according to claim 7, wherein the expression of the proteins expressed by the panel of genes forming the *molecular signature* and by the panel of reference genes is measured with a reagent of detection consisting of labeled monoclonal or polyclonal antibodies against the same.

10. Use of a *molecular signature* consisting of the panel of genes *DEPDC1, CPSF6, C21 orf45, FAM64A, EPB41L4B, NCAPH, WDR67, CENPA, CCNE2, BUB1* in a computer implemented method of breast cancer prognosis based on the expression of these genes.

11. Use of the *molecular signature* according to claim 10, in a computer implemented method of prognosis based on gene expression of the signature in ER+ breast tumors.

12. A kit for a method of breast cancer prognosis on a biopsy sample from a breast cancer patient comprising at least:

   - oligonucleotides for amplifying the 10 genes of the *molecular signature DEPDC1, CPSF6, C21 orf45, FAM64A, EPB41L4B, NCAPH, WDR67, CENPA, CCNE2, BUB1,* and one or more reference genes consisting of at least *ERCC6, ADCY3, TUBGCP2;* or
   - a specific reagent for detecting the expression of proteins expressed from these genes consisting of labeled poly- or monoclonal antibodies;
   - a leaflet of instructions.

13. The kit according to claim 12, wherein the oligonucleotides for amplifying the 10 genes of the *molecular signature* are selected from the oligonucleotides of sequence from SEQ ID NO:6 to SEQ ID NO:25, and the oligonucleotides for one or more reference genes are selected from the oligonucleotides of sequence from SEQ ID NO:26 to SEQ ID NO:31.

**Patentansprüche**

1.  Verfahren zur Brustkrebsprognose, umfassend den Nachweis einer *molekularen Signatur,* bestehend aus der Expression einer Gengruppe, wobei die Gengruppe gebildet wird durch:

    DEPDC1, CPSF6, C21orf45, FAM64A, EPB41L4B, NCAPH, WDR67, CENPA, CCNE2, BUB1 oder den von diesen Genen exprimierten Proteinen, in einer Biopsieprobe, wobei die Überexpression der Gene der *molekularen Signatur* mit einer schlechten Prognose korreliert ist.

2.  Verfahren zur Brustkrebsprognose, basierend den Nachweis einer *molekularen Signatur,* bestehend aus der Expression einer Gengruppe gemäß Anspruch 1, mindestens umfassend die folgenden Schritte:

    - Aufnehmen eines Expressionssignals der Gene DEPDC1, CPSF6, C21orf45, FAM64A, EPB41L4B, NCAPH, WDR67, CENPA, CCNE2, BUB1, die die *molekulare Signatur* bilden,
    - Aufnehmen des Expressionssignals einer Gruppe von Referenzgenen, zumindest bestehend aus ERCC6, ADCY3, TUBGCP2,
    - Einstufen der Probe in die Gruppe einer guten, mittleren oder schlechten Prognose, basierend auf einer Punktzahl, die durch die Summe der Genpunktzahlen ($S_g$) von jedem Gen erhalten wird, die wiederum durch Auswerten der relativen Expression von jedem Gen der *molekularen Signatur* mit der Expression des Referenzgens berechnet werden,

    $$S_f = \sum S_g$$

    wobei ein $S_f$-Wert von weniger als 5 mit einer guten Prognose verbunden ist, ein $S_f$-Wert von weniger als 9 und mehr als 5 mit einer mittleren Prognose verbunden ist und ein $S_f$-Wert von mehr als 9 mit einer schlechten Prognose verbunden ist.

3.  Verfahren zur Brustkrebsprognose gemäß Anspruch 2, wobei die Genpunktzahl ($S_g$) basierend auf der folgenden Formel berechnet wird

$$S_g = \begin{cases} \text{Log}(Exp_l, base=2) - \text{log}(Exp_r, base=2) < -0.5 \implies S_g=0 \\ \begin{aligned}\text{Log}(Exp_l, base=2) - \text{log}(Exp_r, base=2) \geq -0.5 \\ \text{Log}(Exp_l, base=2) - \text{log}(Exp_r, base=2) \leq 0.5\end{aligned} \implies S_g=1 \\ \text{Log}(Exp_l, base=2) - \text{log}(Exp_r, base=2) > 0.5 \implies S_g=2 \end{cases}$$

wobei:

    - $S_g$ gleich 0 ist, wenn ein Gen *I* der *molekularen Signatur* einen Expressionswert $Exp_l$ kleiner als -0,5 auf einer logarithmischen Skala mit der Basis 2 im Vergleich zum entsprechenden Expressionswert Expr des Referenzgens *r* aufweist,
    - $S_g$ gleich 2 ist, wenn ein Gen *I* der *molekularen Signatur* einen Expressionswert $Exp_l$ größer als 0,5 auf einer logarithmischen Skala mit der Basis 2 im Vergleich zum entsprechenden Expressionswert Expr des Referenzgens r aufweist, und
    - $S_g$ gleich 1 ist, wenn ein Gen *I* der *molekularen Signatur* einen Expressionswert $Exp_l$ zwischen weniger als -0,5 und größer als 0,5 auf einer logarithmischen Skala mit der Basis 2 im Vergleich zum entsprechenden Expressionswert Expr des Referenzgens *r* aufweist.

4. Verfahren zur Brustkrebsprognose gemäß Anspruch 2, wobei das Referenzgen für die Gene FAM64A, CENPA, C21orf45, NCAPH das Gen ADCY3 ist, für die Gene BUB1, CCN2, WDR67 das Gen ERCC6 ist und für die Gene CPSF6 und EPB41L4B das Gen TUBGCP2 ist.

5. Verfahren zur Brustkrebsprognose gemäß den Ansprüchen 2 bis 4, wobei die Expression der Gene der *molekularen Signatur* und der Referenzgene mittels molekularer Techniken bestimmt wird, die aus PCR, Mikroarray und Sequenzierung ausgewählt sind.

6. Verfahren zur Brustkrebsprognose gemäß Anspruch 5, wobei zur Bestimmung der Expression der Gene der *molekularen Signatur* und der Referenzgene die Oligonukleotide mit der Sequenz von SEQ ID NO: 6 bis SEQ ID NO: 31 verwendet werden.

7. Verfahren zur Brustkrebsprognose, basierend auf dem Nachweis des Nachweises einer *molekularen Signatur,* die aus der Expression einer Gengruppe gemäß Anspruch 1 besteht, in einer Biopsieprobe, wobei die Genexpression durch die Expression von Proteinen bestimmt wird, wodurch das Verfahren zumindest die folgenden Schritte umfasst:

   - Aufnehmen eines immunhistochemischen Signals der Expression des Proteins, das durch die Gene exprimiert wird,
   - Messen des Signals und Zuordnen eines Zahlenwerts zu der Probe mittels des Quick-Punktzahl-Verfahrens,
   - Einstufen der Probe in die Gruppe der guten oder schlechten Prognose basierend auf einem Punktwert für die Proteinexpression, der niedriger oder höher als 5 ist.

8. Verfahren zur Brustkrebsprognose gemäß Anspruch 7, wobei die Quick-Punktzahl basierend auf den folgenden Schritten berechnet wird:

   - Bewerten der Positivität einer Markierung mit Antikörpern durch Berechnen des Anteils der positiven Zellen und Zuordnen eines Punktwerts von 1 bis 6: 0-4 % = 1, 5-19 % = 2, 20-29 % = 3, 40-59 % = 4, 60-79 % = 5 und 80-100 % = 6,,
   - Nachweisen des aus der Markierung stammenden Signals und Bewerten der mittleren Intensität unter Zuordnung einer Punktzahl von 0 bis 3: kein Signal = 0, schwaches Markierungssignal = 1, mittleres Markierungssignal = 2, starkes Markierungssignal = 3, und
   - Multiplizieren der Punktzahl des Anteils positiver Zellen und des Punktwerts aus der Signalintensität.

9. Verfahren zur Brustkrebsprognose gemäß Anspruch 7, wobei die Expression der Proteine, die durch die Gengruppe exprimiert werden und die *molekulare Signatur* bilden und die durch die Gruppe von Referenzgenen exprimiert werden, mit einem Nachweisreagenz gemessen wird, das aus markierten monoklonalen oder polyklonalen Antikörper gegen diese besteht.

10. Verwendung einer *molekularen Signatur,* bestehend aus der Gengruppe DEPDC1, CPSF6, C21orf45, FAM64A, EPB41L4B, NCAPH, WDR67, CENPA, CCNE2, BUB1 in einem computergestützten Verfahren zur Brustkrebsprognose basierend auf der Expression dieser Gene.

11. Verwendung der *molekularen Signatur* gemäß Anspruch 10, in einem computergestützten Verfahren zur Brustkrebsprognose basierend auf der Genexpression der Signatur in ER+-Brusttumoren.

12. Kit für ein Verfahren zur Brustkrebsprognose an einer Biopsieprobe von einem Brustkrebspatienten, mindestens umfassend:

   - Oligonukleotide zur Amplifizierung der 10 Gene der *molekularen Signatur* DEPDC1, CPSF6, C21orf45, FAM64A, EPB41L4B, NCAPH, WDR67, CENPA, CCNE2, BUB1 und eines oder mehrerer Referenzgene, mindestens bestehend aus ERCC6, ADCY3, TUBGCP2, oder
   - ein spezifisches Reagenz zum Nachweis der Expression von Proteinen, die von diesen Genen exprimiert werden, bestehend aus markierten poly- oder monoklonalen Antikörpern,
   - einen Beipackzettel.

13. Kit, gemäß Anspruch 12, wobei die Oligonukleotide zur Amplifizierung der 10 Gene der *molekularen Signatur* ausgewählt sind aus den Oligonukleotiden der Sequenz von SEQ ID NO: 6 bis SEQ ID NO: 25 und die Oligonukleotide für ein oder mehrere Referenzgene ausgewählt sind aus den Oligonukleotiden der Sequenz von SEQ ID NO: 26 bis SEQ ID NO: 31.

**Revendications**

1. Procédé pour le pronostic du cancer du sein comprenant la détection, dans un échantillon de biopsie, d'une *signature moléculaire* consistant en l'expression d'un panel de gènes formé par : *DEPDC1, CPSF6, C21orf45, FAM64A, EPB41L4B, NCAPH, WDR67, CENPA, CCNE2, BUB1,* ou des protéines exprimées par ceux-ci, dans lequel la surexpression des gènes de *signature moléculaire* est corrélée à un mauvais pronostic.

2. Procédé pour le pronostic du cancer du sein sur la base de la détection d'une *signature moléculaire* consistant en l'expression d'un panel de gènes selon la revendication 1, comprenant au moins les étapes consistant à :

   - acquérir le signal de l'expression des gènes formant la *signature moléculaire DEPDC1, CPSF6, C21orf45, FAM64A, EPB41L4B, NCAPH, WDR67, CENPA, CCNE2, BUB1 ;*
   - acquérir le signal de l'expression d'un panel de gènes de référence constitué d'au moins *ERCC6, ADCY3, TUBGCP2 ;*
   - classer l'échantillon dans le groupe de pronostics bons, intermédiaires ou mauvais sur la base d'un score obtenu par la somme du score de gène ($S_g$) de chaque gène calculé en évaluant l'expression relative de chaque gène de *signature moléculaire* par rapport à l'expression du gène de référence

$$S_f = \Sigma\ S_g$$

   où à une valeur de $S_f$ inférieure à 5 est associé un bon pronostic, à une valeur de $S_f$ inférieure à 9 et supérieure à 5 est associé un pronostic intermédiaire, et à une valeur de $S_f$ supérieure à 9 est associé un mauvais pronostic.

3. Procédé pour le pronostic du cancer du sein selon la revendication 2, dans lequel le score de gène ($S_g$) est calculé sur la base de la formule

$$S_g = \begin{cases} \log(Exp_i, base=2) - \log(Exp_r, base=2) < -0.5 \implies S_g = 0 \\ \begin{aligned} \log(Exp_i, base=2) - \log(Exp_r, base=2) &\geq -0.5 \\ \log(Exp_i, base=2) - \log(Exp_r, base=2) &\leq 0.5 \end{aligned} \implies S_g = 1 \\ \log(Exp_i, base=2) - \log(Exp_r, base=2) > 0.5 \implies S_g = 2 \end{cases}$$

où:

   - $S_g$ est égale à 0 si un gène *I* de la *signature moléculaire* a une valeur d'expression $Exp_i$ inférieure à -0,5 sur l'échelle logarithmique de base 2 par comparaison à la valeur d'expression correspondante $Exp_r$ de son gène de référence $r$,
   - $S_g$ est égale à 2 si un gène *I* de la *signature moléculaire* a une valeur d'expression $Exp_i$ supérieure à 0,5 sur l'échelle logarithmique de base 2 par comparaison à la valeur d'expression correspondante $Exp_r$ de son gène de référence $r$, et
   - $S_g$ est égale à 1 si un gène *I* de la *signature moléculaire* a une valeur d'expression $Exp_i$ intermédiaire entre inférieure à -0,5 et supérieure à 0,5 sur l'échelle logarithmique de base 2 par comparaison à la valeur d'expression correspondante $Exp_r$ de son gène de référence r.

**4.** Procédé pour le pronostic du cancer du sein selon la revendication 2, dans lequel le gène de référence est le gène *ADCY3* pour les gènes *FAM64A, CENPA, C21orf45, NCAPH,* est le gène *ERCC6* pour les gènes *BUB1, CCN2, WDR67,* et est le gène *TUBGCP2* pour les gènes *CPSF6* et *EPB41L4B.*

**5.** Procédé pour le pronostic du cancer du sein selon les revendications 2 à 4, dans lequel l'expression des gènes de *signature moléculaire* et des gènes de référence est déterminée par des techniques moléculaires choisies parmi PCR, microréseau et séquençage.

**6.** Procédé pour le pronostic du cancer du sein selon la revendication 5, dans lequel pour la détermination de l'expression des gènes de *signature moléculaire* et des gènes de référence, les oligonucléotides de séquence de SEQ ID NO:6 à SEQ ID NO:31 sont utilisés.

**7.** Procédé pour le pronostic du cancer du sein sur la base de la détection, dans un échantillon de biopsie, de la détection d'une *signature moléculaire* consistant en l'expression d'un panel de gènes selon la revendication 1, dans lequel l'expression de gène est déterminée par l'expression génique est déterminée par l'expression de protéines, comprenant ainsi au moins les étapes consistant à :

- acquérir un signal immunohistochimique de l'expression de la protéine exprimée par les gènes ;
- mesurer celle-ci et attribuer un score à l'échantillon par l'intermédiaire du procédé Score rapide ;
- classer l'échantillon dans le groupe des pronostics bons ou mauvais sur la base d'un score d'expression protéique inférieur ou supérieur à 5.

**8.** Procédé pour pronostic du cancer du sein selon la revendication 7, dans lequel le score rapide est calculé sur la base des étapes suivantes consistant à :

- évaluer la positivité de marquage avec des anticorps en calculant le pourcentage de cellules positives et en attribuant un score de 1 à 6 : 0 à 4 % = 1 ; 5 à 19 % = 2 ; 20 à 29 % = 3 ; 40 à 59 % = 4 ; 60 à 79 % = 5 et 80 à 100 % = 6 ;
- détecter le signal provenant du marquage et évaluer l'intensité moyenne avec l'attribution d'un score de 0 à 3 : pas de signal = 0 ; signal de marquage faible = 1 ; signal de marquage moyen = 2 ; signal de marquage fort = 3 ; et
- multiplier le score du pourcentage de cellules positives et le score de l'intensité de signal.

**9.** Procédé pour le pronostic du cancer du sein selon la revendication 7, dans lequel l'expression des protéines exprimées par le panel de gènes formant la *signature moléculaire* et par le panel de gènes de référence est mesurée à l'aide d'un réactif de détection constitué d'anticorps polyclonaux ou monoclonaux marqués contre ceux-ci.

**10.** Utilisation d'une *signature moléculaire* consistant en le panel de gènes *DEPDC1, CPSF6, C21orf45, FAM64A, EPB41L4B, NCAPH, WDR67, CENPA, CCNE2, BUB1* dans un procédé mis en oeuvre par ordinateur de pronostic du cancer du sein sur la base de l'expression de ces gènes.

**11.** Utilisation de la *signature moléculaire* selon la revendication 10, dans un procédé implémenté par ordinateur d'un pronostic basé sur l'expression génique de la signature dans les tumeurs du sein ER+.

**12.** Kit pour un procédé de pronostic du cancer du sein sur un échantillon de biopsie provenant d'un patient atteint d'un cancer du sein, comprenant au moins :

- des oligonucléotides pour amplifier les 10 gènes de la *signature moléculaire, DEPDC1, CPSF6, C21orf45, FAM64A, EPB41L4B, NCAPH, WDR67, CENPA, CCNE2, BUB1,* et un ou plusieurs gènes de référence constitués d'au moins ERCC6, ADCY3, TUBGCP2 ; ou
- un réactif spécifique pour détecter l'expression de protéines exprimées par ces gènes de synthèse, consistant en des anticorps polyclonaux ou monoclonaux marqués ;
- un livret d'instructions.

**13.** Kit selon la revendication 12, dans lequel les oligonucléotides pour amplifier les 10 gènes de la *signature moléculaire* sont choisis parmi les oligonucléotides de séquence de SEQ ID NO:6 à SEQ ID NO:25, et les oligonucléotides d'un ou de plusieurs gènes de référence sont choisis parmi les oligonucléotides de séquence de SEQ ID NO:26 à SEQ ID NO:31.

# Figure 1

# Figure 2

## A

## B

## C

# Figure 3

# Figure 4

Figure 5

# Figure 6

# Figure 7

## A

## B

### Cox regression analysis

| | Hazard ratio | P value | 5th percentile | 95th percentile |
| --- | --- | --- | --- | --- |
| Tumour size | 1.022 | 0.030 | 1.002 | 1.042 |
| Tumour stage | 1.498 | 0.001 | 1.201 | 1.869 |
| Lymph-node infiltration | 1.085 | 0.003 | 1.027 | 1.146 |
| Negativity to oestrogen receptor | 1.905 | 0.248 | 0.639 | 5.680 |
| Negativity to HER2 | 0.910 | 0.833 | 0.379 | 2.187 |
| Negativity to progesterone receptor | 1.985 | 0.210 | 0.679 | 5.804 |
| Age | 1.000 | 0.981 | 0.975 | 1.025 |
| Elevated levels of Pin1 | 0.620 | 0.327 | 0.238 | 1.613 |
| Mutations in TP53 | 0.568 | 0.451 | 0.130 | 2.474 |
| Elevated levels of Pin1 and mutations in TP53 | 5.679 | 0.038 | 1.104 | 29.200 |

## C

Cases with elevated levels of Pin1

## D

Cases with low levels of Pin1

# Figure 8

## A

**Pin1 expression levels**

## B

**TP53 State**

## C

### Cox regression analysis
### (Only patients undergoing chemotherapy)

|  | Hazard ratio | P value | 5th percentile | 95th percentile |
|---|---|---|---|---|
| Tumour size | 1.025 | 0.219 | 0.985 | 1.066 |
| Tumour stage | 2.576 | 0.221 | 0.566 | 11.729 |
| Lymph-node infiltration | 1.050 | 0.241 | 0.967 | 1.140 |
| Negativity to oestrogen receptor | 1.487 | 0.604 | 0.332 | 6.670 |
| Negativity to HER2 | 0.697 | 0.533 | 0.224 | 2.168 |
| Negativity to progesterone receptor | 0.820 | 0.045 | 0.676 | 0.996 |
| Age | 0.980 | 0.373 | 0.938 | 1.024 |
| Elevated levels of Pin1 | 0.855 | 0.848 | 0.174 | 4.207 |
| Mutations in TP53 | 0.264 | 0.278 | 0.024 | 2.925 |
| Elevated levels of Pin1 and mutations in TP53 | 16.494 | 0.039 | 1.151 | 236.322 |

55

# Figure 9

Oncogenic signals

Kinase

Inhibition of growth and metastasis supressor genes

Induction of genes promoting an aggressive tumoral phenotype

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2008094678 A
- WO 2005083429 A

- IT PD2011A000201

**Non-patent literature cited in the description**

- **ADORNO, M. ; CORDENONSI, M. ; MONTAGNER, M. ; DUPONT, S. ; WONG, C. ; HANN, B. ; SOLARI, A. ; BOBISSE, S. ; RONDINA, M. B. ; GUZZARDO, V. et al.** A Mutant-p53/Smad Complex Opposes p63 to Empower TGFb-Induced Metastasis. *Cell,* 2009, vol. 137, 87-98
- **ATCHISON, F. W. ; CAPEL, B. ; MEANS, A. R.** Pin1 regulates the timing of mammalian primordial germ cell proliferation. *Development,* 2003, vol. 130, 3579-3586
- **BAO, L. ; KIMZEY, A. ; SAUTER, G. ; SOWADSKI, J. M. ; LU, K. P. ; WANG, D. G.** Prevalent overexpression of prolyl isomerase Pin1 in human cancers. *Am. J. Pathol.,* 2004, vol. 164, 1727-1737
- **BROSH, R. ; ROTTER, V.** When mutants gain new powers: news from the mutant p53 field. *Nat. Rev. Cancer,* 2009, vol. 9, 701-713
- **CAULIN, C. ; NGUYEN, T. ; LANG, G. A. ; GOEPFERT, T. M. ; BRINKLEY, B. R. ; CAI, W. ; LOZANO, G. ; ROOP, D. R.** An inducible mouse model for skin cancer reveals distinct roles for gain- and loss-of-function p53 mutations. *J. Clin. Invest.,* 2007, vol. 117, 1893-1901
- **COUGHLIN, S.S. ; EKWUEME, D.U.** Breast cancer as a global health concern. *Cancer Epidemiol.,* 2009, vol. 33, 315-8
- **DESMEDT, C. ; PIETTE, F. ; LOI, S. ; WANG, Y. ; LALLEMAND, F. ; HAIBE-KAINS, B. ; VIALE, G. ; DE LORENZI, M. ; ZHANG, Y. ; SAGATCHIAN D'ASSIGNIES, M. et al.** Strong Time Dependence of the 76-Gene Prognostic Signature for Node-Negative Breast Cancer Patients in the TRANSBIG Multicenter IndependentValidation Series. *Clin. Cancer Res.,* 2007, vol. 13, 3207-3214
- **DETRE, S. ; SACCANI JOTTI, G. ; DOWSETT, M.** quickscore'' method for immunohistochemical semiquantitation: validation for oestrogen receptor in breast carcinomas. *J. Clin. Pathol.,* 1995, vol. 48, 876-878

- **DROST, J. ; MANTOVANI, F. ; TOCCO, F. ; ELKON, R. ; COMEL, A. ; HOLSTEGE, H. ; KERKHOVEN, R. ; JONKERS, J. ; VOORHOEVE, P.M. ; AGAMI, R.** BRD7 is a candidate tumour suppressor gene required for p53 function. *Nat Cell Biol.,* 2010, vol. 12, 380-9
- **EFEYAN, A. ; MURGA, M. ; MARTINEZ-PASTOR, B. ; ORTEGA-MOLINA, A. ; SORIA, R. ; COLLADO, M. ; FERNANDEZ-CAPETILLO, O. ; SERRANO M.** Limited Role of Murine ATM in Oncogene-Induced Senescence and p53-Dependent Tumor Suppression. *PLoS ONE.,* 2009, vol. 4 (5), e5475
- **GENTLEMAN, R. C. ; CAREY, V. J. ; BATES, D. M. ; BOLSTAD, B. ; DETTLING, M. ; DUDOIT, S. ; ELLIS, B. ; GAULTIER, L.** Bioconductor: open software development for computational biology and bioinformatics. *Genome Biol.,* 2004, vol. 5, R80
- **GUO, A. M. ; COFFILL, C. R. ; LANE, D.** The role of mutant p53 in human cancer. *J of Pathology,* 2011, vol. 223, 116-126
- **IRIZARRY, R. A. ; HOBBS, B. ; COLLIN, F. ; BEAZER-BARCLAY, Y. D. ; ANTONELLIS, K. J. ; SCHERF, U. ; SPEED, T. P.** Exploration, normalization, and summaries of high density oligonucleotide array probe level data. *Biostatistics,* 2003, vol. 4, 249-264
- **JEMAL, A. ; SIEGEL, R. ; WARD, E. ; HAO, Y. ; XU, J. ; THUN, M.J.** Cancer statistics. CA Cancer. *J Clin.,* 2009, vol. 59, 225-49
- **JEMAL, A. ; BRAY, F. ; CENTER, M.M. ; FERLAY, J. ; WARD, E. ; FORMAN, D.** Global cancer statistics. CA Cancer. *J Clin.,* 2011, vol. 61, 69-90
- **LA VECCHIA, C. ; BOSETTI, C. ; LUCCHINI, F. ; BERTUCCIO, P. ; NEGRI, E. ; BOYLE, P. ; LEVI, F.** Cancer mortality in Europe, 2000-2004, and an overview of trends since 1975. *Ann Oncol.,* 2010, vol. 21, 1323-60

- **LANG, G. A. ; IWAKUMA, T. ; SUH, Y. ; LIU, G. ; RAO, A. ; PARANT, J. M. ; VALENTIN-VEGA, Y. A. ; TERZIAN, T. ; CALDWELL, L. C. ; STRONG, L. C. et al.** Gain of Function of a p53 Hot Spot Mutation in a Mouse Model of Li-Fraumeni Syndrome. *Cell,* 2004, vol. 119, 861-872
- **LANGERØD, A. ; ZHAO, H. ; BORGAN, O. ; NESLAND, J. M. ; BUKHOLM, I. R. K. ; IKDAHL, T. ; KÅRESEN, R. ; BØRRESEN-DALE, A. L. ; JEFFREY, S. S.** TP53 mutation status and gene expression profiles are powerful prognostic markers of breast cancer. *Breast Cancer Res.,* 2007, vol. 9, R30
- **LU, K. P. ; ZHOU, X. Z.** The prolyl isomerase PIN1: a pivotal new twist in phosphorylation signalling and disease. *Nat. Rev. Mol. Cell Biol.,* 2007, vol. 8, 904-916
- **MALKIN, D. ; LI, F. P. ; STRONG, L. C. ; FRAUMENI, J. F. JR. ; NELSON, C. E. ; KIM, D. H. ; KASSEL, J. ; GRYKA, M. A. ; BISCHOFF, F. Z. ; TAINSKY, M. A. et al.** Germ line p53 mutations in a familial syndrome of breast cancer, sarcomas, and other neoplasms. *Science,* 1990, vol. 250, 1233-1238
- **MANTOVANI, F. ; TOCCO, F. ; GIRARDINI, J. E. ; SMITH, P. ; GASCO, M. ; LU, X. ; CROOK, T. ; DEL SAL, G.** The prolyl isomerase Pin1 orchestrates p53 acetylation and dissociation from the apoptosis inhibitor iASPP. *Nat. Struct. Mol. Biol.,* 2007, vol. 14, 912-920
- **MILLER, L. D. ; SMEDS, J. ; GEORGE, J. ; VEGA, V. B. ; VERGARA, L. ; PLONER, A. ; PAWITAN, Y. ; HALL, P. ; KLAAR, S. ; LIU, E. T. et al.** An expression signature for p53 status in human breast cancer predicts mutation status, transcriptional effects, and patient survival. *Proc. Natl. Acad. Sci. USA.,* 2005, vol. 102, 13550-13555
- **MULLER, P. ; CASWELL, P. T. ; DOYLE, B. ; IWANICKI, M. P. ; TAN, E. H. ; KARIM, S. ; LUKASHCHUK, N. ; GILLESPIE, D. A. ; LUDWIG, R. L. ; GOSSELIN, P. et al.** Mutant p53 Drives Invasion by Promoting Integrin Recycling. *Cell,* 2009, vol. 139, 1327-1341
- **OLIVIER, M. ; LANGERØD, A. ; CARRIERI, P. ; BERGH, J. ; KLAAR, S. ; EYFJORD, J. ; THEILLET, C. ; RODRIGUEZ, C. ; LIDEREAU, R. ; BIECHE, I. et al.** The Clinical Value of Somatic TP53 GeneMutations in 1,794 Patients with Breast Cancer. *Clin. Cancer. Res.,* 2006, vol. 12, 1157-1167
- **PAWITAN, Y. ; BJÖHLE, J. ; AMLER, L. ; BORG, A. L. ; EGYHAZI, S. ; HALL, P. ; HAN, X. ; HOLMBERG, L. ; HUANG, F. ; KLAAR, S. et al.** Gene expression profiling spares early breast cancer patients from adjuvant therapy: derived and validated in two population-based cohorts. *Breast Cancer Res.,* 2005, vol. 7, R953-964
- **PRAT, A. ; PEROU, C.M.** Deconstructing the molecular portraits of breast cancer. *Mol Oncol.,* 2011, vol. 5, 5-23
- **RUSTIGHI, A. ; TIBERI, L. ; SOLDANO, A. ; NAPOLI, M. ; NUCIFORO, P. ; ROSATO, A. ; KAPLAN, F. ; CAPOBIANCO, A. ; PECE, S. ; DI FIORE, P. P. et al.** The prolyl-isomerase Pin1 is a Notch1 target that enhances Notch1 activation in cancer. *Nat. Cell Biol.,* 2009, vol. 11, 133-142
- **RYO, A. ; LIOU, Y. C. ; WULF, G. ; NAKAMURA, M. ; LEE, S. W. ; LU, K. P.** PIN1 Is an E2F Target Gene Essential for Neu/Ras-Induced Transformation of Mammary Epithelial. *Cells. Mol. Cell. Biol.,* 2002, vol. 22, 5281-5295
- **SMYTH, G. K.** Linear models and empirical bayes methods for assessing diferential expression in microarray experiments. *Stat Appl Genet Mol Biol,* 2004, vol. 3
- **SONG, H. ; HOLLSTEIN, M. ; XU, Y.** p53 gain-of-function cancer mutants induce genetic instability by inactivating ATM. *Nature Cell Biology,* 2007, vol. 9, 573-580
- **SOTIRIOU, C. ; WIRAPATI, P. ; LOI, S. ; HARRIS, A. ; FOX, S. ; SMEDS, J. ; NORDGREN, H. ; FARMER, P. ; PRAZ, V. ; HAIBE-KAINS, B. et al.** Gene expression profiling in breast cancer: understanding the molecular basis of histologic grade to improve prognosis. *J. Natl. Cancer Inst.,* 2006, vol. 98, 262-272
- **SOUSSI, T. ; BEROUD, C.** Assessing TP53 status in human tumours to evaluate clinical outcome. *Nature Reviews on Cancer,* 2001, vol. 1, 233-240
- **SOUSSI, T. ; WIMAN, K. G.** Shaping Genetic Alterations in Human Cancer: The p53 Mutation Paradigm. *Cancer Cell,* 2007, vol. 12, 303-312
- **TERZIAN, T. ; SUH, Y. ; IWAKUMA, T. ; POST, S. M. ; NEUMANN, M. ; LANG, G. A. ; VAN PELT, C. S. ; LOZANO, G.** The inherent instability of mutant p53 is alleviated by Mdm2 or p16INK4a loss. *Genes & Dev.,* 2008, vol. 22, 1337-1344
- UKCCCR (1989). UKCCCR guidelines for the welfare of animals in experimental neoplasia. *Cancer Metastasis Rev.,* vol. 8, 82-88
- **VAN'T VEER, L. J. ; DAI, H. ; VAN DE VIJVER, M. J. ; HE, Y. D. ; HART, A. A. ; MAO, M. ; PETERSE, H. L. ; VAN DER KOOY, K. ; MARTON, M. J. ; WITEVEEN, A. T. et al.** Gene expression profiling predicts clinical outcome of breast cancer. *Nature,* 2002, vol. 415, 530-536
- **WULF, G. ; GARG, P. ; LIOU, Y. C. ; IGLEHART, D. ; LU, K. P.** Modeling breast cancer in vivo and ex vivo reveals an essential role of Pin1 in tumorigenesis. *EMBO J.,* 2004, vol. 23, 3397-3407
- **YEH, E. ; MEANS, A. R.** PIN1, the cell cycle and cancer. *Nat. Rev. Cancer,* 2007, vol. 7, 381-388
- **ZACCHI, P. ; GOSTISSA, M. ; UCHIDA, T. ; SALVAGNO, C. ; AVOLIO, F. ; VOLINIAK, S. ; RONAI, Z. ; BLANDINO, G. ; SCHNEIDER, C. ; DEL SAL, G.** The prolyl isomerase Pin1 reveals a mechanism to control p53 functions after genotoxic insults. *Nature,* 2002, vol. 419, 853-857

- **ZHENG, H. ; YOU, H. ; ZHOU, X. Z. ; MURRAY, S. A. ; UCHIDA, T. ; WULF, G. ; TANG, X. ; LU, K. P. ; XIAO, Z.-X.** The Prolyl isomerase Pin1 is a novel regulator of p53 in genotoxic response. *Nature,* 2002, vol. 419, 849-853

SEQUENCE LISTING

<110> UNIVERSITA' DEGLI STUDI DI TRIESTE UNIVERSITY OF DUNDEE

<120> Prognostic method for breast tumours based on expression of the prolyl isomerase enzyme Pin1 combined with identification of the presence of

missense mutations in the gene TP53

<130> 10945PTWO

<150> ITPD2011A000201
<151> 2011-06-16

<160> 58

<170> BiSSAP 1.0

<210> 1
<211> 27
<212> DNA
<213> Homo sapiens

<220>
<221> source
<222> 1..27
<223> /mol_type="DNA" /organism="Homo sapiens"

<400> 1
aatgtcagtc tgagtcaggc ccttctg 27

<210> 2
<211> 26
<212> DNA
<213> Homo sapiens

<220>
<221> source
<222> 1..26
<223> /mol_type="DNA" /organism="Homo sapiens"

<400> 2
tgatgctgtc cccggacgat attgaa 26

<210> 3
<211> 24
<212> DNA
<213> Homo sapiens

<220>
<221> source
<222> 1..24
<223> /mol_type="DNA" /organism="Homo sapiens"

<400> 3
tctcccagga caggcacaaa cacg        24


<210> 4
<211> 22
<212> DNA
<213> Homo sapiens


<220>
<221> source
<222> 1..22
<223> /mol_type="DNA" /organism="Homo sapiens"


<400> 4
tttgcgtgtg gagtatttgg at 22


<210> 5
<211> 24
<212> DNA
<213> Homo sapiens


<220>
<221> source
<222> 1..24
<223> /mol_type="DNA" /organism="Homo sapiens"


<400> 5
ttggcaaaac atcttgttga gggc 24


<210> 6
<211> 23
<212> DNA
<213> Homo sapiens


<220>
<221> source
<222> 1..23
<223> /mol_type="DNA" /organism="Homo sapiens"


<400> 6
attcaagcca cagagtggag cag 23


<210> 7
<211> 26
<212> DNA
<213> Homo sapiens


<220>
<221> source
<222> 1..26
<223> /mol_type="DNA" /organism="Homo sapiens"


<400> 7
agaacttgtg ttggcaacct tatgtg 26


<210> 8
<211> 20
<212> DNA

<213> Homo sapiens

<220>
<221> source
<222> 1..20
<223> /mol_type="DNA" /organism="Homo sapiens"

<400> 8
gcgactcgct gagctgggtg 20

<210> 9
<211> 20
<212> DNA
<213> Homo sapiens

<220>
<221> source
<222> 1..20
<223> /mol_type="DNA" /organism="Homo sapiens"

<400> 9
ccccgcgcag cacaaagtct 20

<210> 10
<211> 20
<212> DNA
<213> Homo sapiens

<220>
<221> source
<222> 1..20
<223> /mol_type="DNA" /organism="Homo sapiens"

<400> 10
tgagccgagc ggtagctggt 20

<210> 11
<211> 20
<212> DNA
<213> Homo sapiens

<220>
<221> source
<222> 1..20
<223> /mol_type="DNA" /organism="Homo sapiens"

<400> 11
gggctggggc tgctgcttag 20

<210> 12
<211> 22
<212> DNA
<213> Homo sapiens

<220>
<221> source
<222> 1..22
<223> /mol_type="DNA" /organism="Homo sapiens"

<400> 12

cttcctccca tcaacacagt cg 22

<210> 13
<211> 22
<212> DNA
<213> Homo sapiens

<220>
<221> source
<222> 1..22
<223> /mol_type="DNA" /organism="Homo sapiens"

<400> 13

tgcttctgct gcctcttgta gg 22

<210> 14
<211> 20
<212> DNA
<213> Homo sapiens

<220>
<221> source
<222> 1..20
<223> /mol_type="DNA" /organism="Homo sapiens"

<400> 14

aggggctgtt cctggtgggg 20

<210> 15
<211> 21
<212> DNA
<213> Homo sapiens

<220>
<221> source
<222> 1..21
<223> /mol_type="DNA" /organism="Homo sapiens"

<400> 15

ggcccagcta gaggaggagg c 21

<210> 16
<211> 26
<212> DNA
<213> Homo sapiens

<220>
<221> source
<222> 1..26
<223> /mol_type="DNA" /organism="Homo sapiens"

<400> 16

tgggtattat ctgccatgaa gtgcct 26

<210> 17
<211> 23
<212> DNA

EP 2 721 178 B1

<213> Homo sapiens

<220>
<221> source
<222> 1..23
<223> /mol_type="DNA" /organism="Homo sapiens"

<400> 17
aggttgcagc aagcccaaaa tgt 23

<210> 18
<211> 20
<212> DNA
<213> Homo sapiens

<220>
<221> source
<222> 1..20
<223> /mol_type="DNA" /organism="Homo sapiens"

<400> 18
cgacgggacc gaagtgagcg 20

<210> 19
<211> 20
<212> DNA
<213> Homo sapiens

<220>
<221> source
<222> 1..20
<223> /mol_type="DNA" /organism="Homo sapiens"

<400> 19
cagtgcgcaa cctgggcaga 20

<210> 20
<211> 20
<212> DNA
<213> Homo sapiens

<220>
<221> source
<222> 1..20
<223> /mol_type="DNA" /organism="Homo sapiens"

<400> 20
ctccaggctg cagctcgctc 20

<210> 21
<211> 20
<212> DNA
<213> Homo sapiens

<220>
<221> source
<222> 1..20
<223> /mol_type="DNA" /organism="Homo sapiens"

<400> 21
cagccgggtg ctcttctggc 20


<210> 22
<211> 20
<212> DNA
<213> Homo sapiens


<220>
<221> source
<222> 1..20
<223> /mol_type="DNA" /organism="Homo sapiens"


<400> 22
gaggagcctg ccccctgtca 20


<210> 23
<211> 20
<212> DNA
<213> Homo sapiens


<220>
<221> source
<222> 1..20
<223> /mol_type="DNA" /organism="Homo sapiens"


<400> 23
tgggcctcct gctgctgact 20


<210> 24
<211> 20
<212> DNA
<213> Homo sapiens


<220>
<221> source
<222> 1..20
<223> /mol_type="DNA" /organism="Homo sapiens"


<400> 24
aggcaacaag gagagcggca 20


<210> 25
<211> 20
<212> DNA
<213> Homo sapiens


<220>
<221> source
<222> 1..20
<223> /mol_type="DNA" /organism="Homo sapiens"


<400> 25
agcagtcgcc tgtgccatca 20


<210> 26
<211> 23
<212> DNA

<213> Homo sapiens

<220>
<221> source
<222> 1..23
<223> /mol_type="DNA" /organism="Homo sapiens"

<400> 26
tctctgtttc cttgtgggcg ctc 23

<210> 27
<211> 22
<212> DNA
<213> Homo sapiens

<220>
<221> source
<222> 1..22
<223> /mol_type="DNA" /organism="Homo sapiens"

<400> 27
caccccacag cagaggtgga ca 22

<210> 28
<211> 22
<212> DNA
<213> Homo sapiens

<220>
<221> source
<222> 1..22
<223> /mol_type="DNA" /organism="Homo sapiens"

<400> 28
gtgggctggc aggtcttctt tg 22

<210> 29
<211> 22
<212> DNA
<213> Homo sapiens

<220>
<221> source
<222> 1..22
<223> /mol_type="DNA" /organism="Homo sapiens"

<400> 29
ggtgcttgcg gtcagccatg ta 22

<210> 30
<211> 22
<212> DNA
<213> Homo sapiens

<220>
<221> source
<222> 1..22
<223> /mol_type="DNA" /organism="Homo sapiens"

<400> 30
gacgtcacgc tcacttccgc cg 22

<210> 31
<211> 22
<212> DNA
<213> Homo sapiens

<220>
<221> source
<222> 1..22
<223> /mol_type="DNA" /organism="Homo sapiens"

<400> 31
tcagccccat ctcctccgtg ga 22

<210> 32
<211> 19
<212> RNA
<213> Homo sapiens

<220>
<221> source
<222> 1..19
<223> /mol_type="RNA" /organism="Homo sapiens"

<400> 32
cgggagagga ggacuuuga 19

<210> 33
<211> 19
<212> RNA
<213> Homo sapiens

<220>
<221> source
<222> 1..19
<223> /mol_type="RNA" /organism="Homo sapiens"

<400> 33
gccauuugaa gacgccucg 19

<210> 34
<211> 19
<212> RNA
<213> Homo sapiens

<220>
<221> source
<222> 1..19
<223> /mol_type="RNA" /organism="Homo sapiens"

<400> 34
gacuccagug guaaucuac 19

<210> 35
<211> 19
<212> RNA

<213> Homo sapiens

<220>
<221> source
<222> 1..19
<223> /mol_type="RNA" /organism="Homo sapiens"

<400> 35
ggugaaccuu aguaccuaa 19

<210> 36
<211> 19
<212> RNA
<213> Homo sapiens

<220>
<221> source
<222> 1..19
<223> /mol_type="RNA" /organism="Homo sapiens"

<400> 36
gugaccagcg aauaccugu 19

<210> 37
<211> 20
<212> DNA
<213> Homo sapiens

<220>
<221> source
<222> 1..20
<223> /mol_type="DNA" /organism="Homo sapiens"

<400> 37
gggctgcctc acacccgttt 20

<210> 38
<211> 20
<212> DNA
<213> Homo sapiens

<220>
<221> source
<222> 1..20
<223> /mol_type="DNA" /organism="Homo sapiens"

<400> 38
acacctgccc ttccagtggg 20

<210> 39
<211> 22
<212> DNA
<213> Homo sapiens

<220>
<221> source
<222> 1..22
<223> /mol_type="DNA" /organism="Homo sapiens"

```
<400> 39
ggtgctgagc agctggtgca aa 22


<210> 40
<211> 22
<212> DNA
<213> Homo sapiens


<220>
<221> source
<222> 1..22
<223> /mol_type="DNA" /organism="Homo sapiens"


<400> 40
tgcggggtca cattactccc ct 22


<210> 41
<211> 20
<212> DNA
<213> Homo sapiens


<220>
<221> source
<222> 1..20
<223> /mol_type="DNA" /organism="Homo sapiens"


<400> 41
aaatccagga gttgcagtgg 20


<210> 42
<211> 20
<212> DNA
<213> Homo sapiens


<220>
<221> source
<222> 1..20
<223> /mol_type="DNA" /organism="Homo sapiens"


<400> 42
actctcaggg gctccttctc 20


<210> 43
<211> 23
<212> DNA
<213> Homo sapiens


<220>
<221> source
<222> 1..23
<223> /mol_type="DNA" /organism="Homo sapiens"


<400> 43
acttcccaag ggtcacacag tca        23


<210> 44
<211> 21
<212> DNA
```

<213> Homo sapiens

<220>
<221> source
<222> 1..21
<223> /mol_type="DNA" /organism="Homo sapiens"

<400> 44
tgtggcagaa tgggacaggc g 21

<210> 45
<211> 24
<212> DNA
<213> Homo sapiens

<220>
<221> source
<222> 1..24
<223> /mol_type="DNA" /organism="Homo sapiens"

<400> 45
tggcaatgaa tgcgagagga aggt 24

<210> 46
<211> 21
<212> DNA
<213> Homo sapiens

<220>
<221> source
<222> 1..21
<223> /mol_type="DNA" /organism="Homo sapiens"

<400> 46
ccccgggcat tctgtgaacg g 21

<210> 47
<211> 21
<212> DNA
<213> Homo sapiens

<220>
<221> source
<222> 1..21
<223> /mol_type="DNA" /organism="Homo sapiens"

<400> 47
agggccaggc agaaaaaccg t 21

<210> 48
<211> 20
<212> DNA
<213> Homo sapiens

<220>
<221> source
<222> 1..20
<223> /mol_type="DNA" /organism="Homo sapiens"

<400> 48
cgggtttccc tggcgctgtt 20

<210> 49
<211> 20
<212> DNA
<213> Homo sapiens

<220>
<221> source
<222> 1..20
<223> /mol_type="DNA" /organism="Homo sapiens"

<400> 49
ggcttgggac gcctcacgat 20

<210> 50
<211> 23
<212> DNA
<213> Homo sapiens

<220>
<221> source
<222> 1..23
<223> /mol_type="DNA" /organism="Homo sapiens"

<400> 50
gcccatgaac tgcctagctg tgg 23

<210> 51
<211> 21
<212> DNA
<213> Homo sapiens

<220>
<221> source
<222> 1..21
<223> /mol_type="DNA" /organism="Homo sapiens"

<400> 51
agtgcagcag tgagacgggg a 21

<210> 52
<211> 22
<212> DNA
<213> Homo sapiens

<220>
<221> source
<222> 1..22
<223> /mol_type="DNA" /organism="Homo sapiens"

<400> 52
accccagagg ctagtccgaa gg 22

<210> 53
<211> 20
<212> DNA

<213> Homo sapiens

<220>
<221> source
<222> 1..20
<223> /mol_type="DNA" /organism="Homo sapiens"

<400> 53
atcggggcgt ctggtgggaa 20

<210> 54
<211> 20
<212> DNA
<213> Homo sapiens

<220>
<221> source
<222> 1..20
<223> /mol_type="DNA" /organism="Homo sapiens"

<400> 54
cgaggagggg aggcagggtc 20

<210> 55
<211> 20
<212> DNA
<213> Homo sapiens

<220>
<221> source
<222> 1..20
<223> /mol_type="DNA" /organism="Homo sapiens"

<400> 55
ccctgcctgc atctgtgcca 20

<210> 56
<211> 23
<212> DNA
<213> Homo sapiens

<220>
<221> source
<222> 1..23
<223> /mol_type="DNA" /organism="Homo sapiens"

<400> 56
gcaggcaaag aggttgcctt tgt 23

<210> 57
<211> 20
<212> DNA
<213> Homo sapiens

<220>
<221> source
<222> 1..20
<223> /mol_type="DNA" /organism="Homo sapiens"

<400> 57
cacgaggctc ccaaagggcg 20

<210> 58
<211> 20
<212> DNA
<213> Homo sapiens

<220>
<221> source
<222> 1..20
<223> /mol_type="DNA" /organism="Homo sapiens"

<400> 58
aaaatccgcc caagcggcca 20